(19) **Europäisches Patentamt**

**European Patent Office**

**Office européen des brevets**

(11) **EP 1 216 076 B1**

(12) **EUROPEAN PATENT SPECIFICATION**

(45) Date of publication and mention
of the grant of the patent:
**16.08.2006 Bulletin 2006/33**

(51) Int Cl.:
***A61N 2/04*** (2006.01)

(21) Application number: **00956141.6**

(22) Date of filing: **28.08.2000**

(86) International application number:
**PCT/DK2000/000474**

(87) International publication number:
**WO 2001/015774 (08.03.2001 Gazette 2001/10)**

(54) **A METHOD AND AN APPARATUS FOR STIMULATING/MODULATING BIOCHEMICAL PROCESSES USING PULSED ELECTROMAGNETIC FIELDS**

EINE VORRICHTUNG UND EINE METHODE ZUR ANREGUNG/MODULATION BIOCHEMISCHER PROZESSER DURCH PULSIERENDE ELEKTROMAGNETISCHE FELDER VON MEHRFACHSPULEN

PROCEDE ET APPAREIL PERMETTANT DE STIMULER/MODULER DES PROCESSUS BIOLOGIQUES AU MOYEN DE CHAMPS ELECTROMAGNETIQUES PULSES

(84) Designated Contracting States:
**AT BE CH CY DE DK ES FI FR GB GR IE IT LI LU MC NL PT SE**

(30) Priority: **31.08.1999 DK 120799**

(43) Date of publication of application:
**26.06.2002 Bulletin 2002/26**

(73) Proprietor: **Biofields APS**
**1602 Copenhagen V (DK)**

(72) Inventors:
• **DISSING, Steen**
**DK-2920 Charlottenlund (DK)**
• **UNDEN, Mogens**
**DK-3100 Hornbaek (DK)**

• **LARSEN, Teddy, Hebo**
**DK-2550 Hvidovre (DK)**
• **SCHOU, Soren**
**DK-2840 Holte (DK)**
• **PETERSEN, Hans, Nissen**
**DK-3540 Lynge (DK)**

(74) Representative: **Plougmann & Vingtoft A/S**
**Sundkrogsgade 9**
**P.O. Box 831**
**2100 Copenhagen OE (DK)**

(56) References cited:
**EP-A- 0 709 115        EP-A- 0 940 157**
**WO-A-85/00293        WO-A-99/10041**
**DE-A- 3 403 786        DE-A- 19 914 762**
**US-A- 5 518 496**

**Description**

**FIELD OF THE INVENTION**

The present application relates to a method and an apparatus for stimulating and/or modulating growth and differentiation in biological or plant tissue, seeds, plants, and micro organisms. An apparatus of this type comprises a pulse generator and a plurality of coils, in which pulsed currents cause fluctuating magnetic fields in a predetermined region holding the material to be stimulated. The fluctuating magnetic fields will induce an electric field in the material.

**BACKGROUND OF THE INVENTION**

Pulsed electromagnetic fields (PEMF) have been used widely to treat delayed non-healing fractures, pseudoarthrosis, osteoarthritis, bone fractures and related problems (Bassett, C.A., Mitchell, S.N. & Gaston, S.R. (1981); (Trock et al., 1994). "Treatment of ununited tibial diaphyseal fractures with pulsing electromagnetic fields", *Journal of Bone and Joint Surgery [American],* 63-A, 511-523 and Bassett, C.A.L., Pilla, A.A. & Pawluk, R.J. (1977) "A non-operative salvage of surgically-resistant pseudarthroses and non-unions by pulsing electromagnetic fields: a preliminary report", *Clinical Orthopaedics,* 128-143) and have also been suggested for the treatment of nerve growth and wound healing (Sisken, B.F., Kanje, M., Lundborg, G., Herbst, E. & Kurtz, W. (1989), "Stimulation of rat sciatic nerve regeneration with pulsed electromagnetic fields", *Brain Research,* 485, 309-316 and Patino, O., Grana, D., Bolgiani, A., Prezzavento, G., Mino, J., Merlo, A. & Benaim, F. (1996), and "Pulsed electromagnetic fields in experimental cutaneous wound healing in rats" *Journal of Burn Care and Rehabilitation,* 17, 528-531). It has been suggested that some of the important effects relating to an enhanced bone growth is the PEMF-induced promotion of angiogenesis, but this issue is not yet resolved (The National Institute of Environmental Health Services (NIEHS) : "Assessment of Health Effects from Exposure to Power-Line Frequency Electric and Magnetic Fields". (http://www.niehs.nih.gov/emfrapid/home.htm)).

A temporarily varying magnetic flux through an area induces an electric field, E, along the perimeter of the area according to the basic laws of electromagnetism. If the varying magnetic field, B(t), is applied to a material containing free (or mobile) charge carriers, these will be accelerated by the electric field thereby generating eddy currents in the material. The induced electric field or the generated current depends upon the rate of change, $dB/dt$, of the magnetic field, the electric field or current increasing with increasing rate of change.

The main point in treating biological tissue i.e. bone healing, wound healing, nerve growth, and angiogenesis is the introduction of tissue currents with an intensity and duration that can activate cellular signalling processes and extracellular signals, thus initiating cell proliferation and differentiation and other biological processes.

WO 85/00293 and WO 99/10041 describe the use of conducting coils to stimulate growth and healing in living tissue. The coils are positioned so as to generate a strong field at the region to be treated and a pulsating current signal is supplied for conduction in the coils.

PEMF has been used for activating muscle and neural cells as an alternative method to electrodes see e.g. EP 788 813 or US 5,738,625. PEMF induced by conducting coils has the advantage that no electrodes in direct contact with the skin have to be used. The PEMF used for activating cells must be fast and/or large enough to induce electric potentials large enough to elicit the action potentials of excitable cells. In order to achieve such large electrical potentials, very large currents are used in the coil and the fields from several coils are added. In EP 788 813, the PEMF is used to activate muscle cells to flex a group of pelvic floor muscles in order to treat urinary incontinence. In US 5,738,625, the PEMF is used to activate neural cells in order to investigate or diagnose the nervous system.

The effects of fluctuating magnetic fields in the tissue can be anticipated to be due to the effect of the induced electric field upon charged particles and entities (ions, molecules and macromolecules such as proteins, and inositol phosphates and other signalling compounds, cells and their extracellular signalling compounds such as hormones and other neurotransmitters etc.). Hence, the effects of fluctuating magnetic fields can be anticipated to be due to the extracellular as well as intracellular events caused by the electric fields and currents.

Regarding extracellular effects it can be expected that the on and off rate constants for the associations between neurotransmitters, hormones and their receptors will be affected, to the extent that net positive or negative charges are associated with the process, as well as inducing piezo-electric currents in bone tissue, thus mimicking physiological processes. The intracellular effects that might be the most affected are biochemical reactions that are involved in promoting cell division and differentiation. Amongst cellular signalling processes that have been suggested to be essential for the initiation of cell proliferation is the activation of protein kinase A. This enzyme is activated by cAMP (cyclic adenosine 5'monophophate) that is synthesised from ATP by a receptor activated adenylyl cyclase. cAMP binds to protein kinase A and forms catalytic subunits, and this signal can be carried to the nucleus. Here it leads to activation of cAMP-inducible genes. Activation of the synthesis of cGMP, by iron containing enzymes such as nitrogen oxide synthetase that in turn activates classes of protein kinase G, are also important candidates. Several studies have implicated that the activation of omithine decarboxylase, causing synthesis of prutescine and other related compounds, that promotes DNA transcription, also appear to be essential. The mechanism by which signalling processes are initiated appear to be due to a combined effect of proteins with a net charge, that will move in the cell interior (G-proteins, protein kinases, mRNA binding proteins etc.). Those can associate with target proteins and exert a biological effect and changes in the association constants for these processes will affect cellular function. Other ions, such as $Ca^{2+}$, are highly affected by electrical fields, and will also exert a biological effect by associating with intracellular proteins and ion channels. The essential point is that signalling molecules with net charges or areas with net charges will be affected by the changing magnetic fields and all charged particles will rotate in magnetic fields depending on movements relative to the magnetic field.

One important aspect of promoting growth of osteoblasts, chondroblasts, chondrocytes and their derivatives (bone and cartilage), nerve cells, and other tissues is the induction of growth of small vessels (capillaries) that supply the blood cells, hormones and nutrients for sustaining cell proliferation and differentiation. The small vessels consist of endothelial cells, smooth muscle cells, and other cell types that together will protrude into new areas following the activation of nitric oxide (NO) and growth factors. These cells are also connected to each other both through signalling by chemical substances but also electrically through gap junctions. Both NO, vascular endothelial growth factor (VEGF) and other factors, appear to play an essential role in activating growth and differentiation amongst other things through activation of MAP kinase signalling pathways. However, the intracellular signalling processes play an equally important role in the cellular activation and when considering the effects of PEMF on angiogenesis both extracellular as well intracellular events should be considered.

The induced electric field from a circular coil can be calculated in a plane parallel to the coil, at a given distance from the coil. Due to the cylindrical symmetry, the induced electric field will have a circular symmetry in the plane, and have a maximum at a circle centred at the centre axis of the coil with a radius, r, smaller than the radius, R, of the coil. As the distance from the coil to the plane increases, the peak of the maximum flattens out and the radius of the ring shaped maximum varies slightly. Thereby, the maximum of the induced electrical field in a direction away from the coil, form a tubular region centred on the centre axis of the coil, and in a plane at a given distance from the coil, the induced electrical field will have a ring shaped maximum with a minimum in the centre.

In the apparatuses of the prior art, the to be treated is centred in the coil thereby experiencing an approximately homogeneous induced electrical field while the ring shaped maximum is positioned in regions encircling the region to be treated. Hence all-over, the field is inhomogeneous.

The biochemical features outlined in the above take place at a large range of electric fields. However, if the induced electrical field gets too high in the region to be treated, it will lead to elicitation of action potentials of excitable cells in the region. Elicitation of cellular action potentials is normally undesirable since it may lead to nuisance for the patient or give rise to undesirable physiological reactions. For example, the effects of the large induced electrical fields in EP 788 813 or US 5,738,625 are a flexing of muscles due to activation of muscle cells or elicitation of nerve impulses due to activation of neural cells. These are undesirable side effects for a person undergoing a continuous treatment.

Therefore, under normal conditions it is not possible simply to increase the current or its rise time in the coils in order to achieve a larger induced electrical field over the region to be treated. The average field can only be increased until the field at the ring shaped maximum reaches the limit for elicitation of the action potentials of cells. Thus the average induced electrical field in the larger central part will not be increased to a very high degree. Hence by increasing the current or its rise time, the field in the region to be treated can only be brought to an average value lying considerably lower than the limit for elicitation of the action potentials of cells. In order to maintain the homogeneous field in the region to be treated, one will pay the price of a lower field and a large stimulation in the surrounding regions, which are not to be treated.

DE-A-34 03 786 discloses a honeycomb structured coil arrangement for magnetotherapy. Subgroups of the coils are cyclically activated so as to create a rotating magnetic field that provides for therapy of deep tissue layers.

SUMMARY OF THE INVENTION

The present invention utilises the realisation that stimulation of the biological tissue depends on the magnetic field in a way not previously anticipated. According to investigations performed by the inventors, there is an improved stimulation of the biological tissue in regions lying above or below the perimeter of the coil and not in the regions lying above the centre of the coil. Also, the investigations showed that a continuous treatment for longer periods of time (few hours to several days) is often desirable. Hence the efficiency of the biochemical features outlined in the above and giving rise to treatment of biological tissue i.e. bone healing, wound healing, nerve growth and angiogenesis, depends on the induced electrical fields. In order to optimise the effects in the region to be treated, it is desirable to increase the induced electric field in this region and to have a constant average field over the region.

Especially, it has been found that larger B- and /or E-field gradients seem to have a positive effect on the cells, tissue and micro organisms. Such gradients may especially be formed by two coils oppositely polarised and positioned adjacently in relation to the cells. In this manner, especially in the intermediate area of the fields of the coils a larger field gradient is obtained. This effect has not been described hitherto.

The present technique imposes movement of ions and proteins in the tissue from all germinal layers that affects cellular activity in individual cells and in biological tissue as a whole. Important factors are the magnitude of the driving force (through imposing a changing magnetic field strength) with the direction of the magnetic field vectors; the frequency and shape of the pulses and the electrical potential. Those factors determine to which extent a compound possessing a net charge (ions, macromolecules etc.) are affected in a way such that a biological process (proliferation and differentiation) is initiated. The energy level by which the tissue is affected preferably does not cause significant changes in membrane potential and does not evoke action potentials in excitable tissues.

The invention relates to an apparatus as defined in claim 1 and to a method as defined in claim 16.

Thus, by providing honeycomb structured coils where the desired E-field gradients are provided, a better influence on the tissue/cells/micro organisms is provided. It should be noted that, naturally, a given coil is a member of a honeycomb structures in that it will have three adjacent coils in the apparatus.

In the present context, the centre axis of a coil is a symmetry axis normally directed along the central axis of a tubular coil or perpendicularly (positioned centrally) to a plane of a flat coil.

According to the invention first coil of each honeycomb structure is adapted to conduct the current pulse in a clockwise direction and wherein three of the nearest neighbouring coils of the first coil are adapted to conduct the current pulse in a counter clock-wise direction taken along the centre axes of the coils in a direction toward the cells, micro organisms and/or tissue.

Naturally, the number of honeycomb structures will depend on the actual use of the apparatus - and on the size thereof.

A cross section of each coil, perpendicularly to the centre axis, may be at the most 100 cm$^2$, such as at the most 50 cm$^2$, preferably at the most 25 cm$^2$, such as at the most 10 cm$^2$, such as at the most 9 cm$^2$, preferably at the most 8 cm$^2$, such as at the most 7 cm$^2$, preferably at the most 6 cm$^2$, such as at the most 5 cm$^2$, preferably at the most 4 cm$^2$, such as at the most 3 cm$^2$, preferably at the most 2 cm$^2$, such as at the most 1 cm$^2$, preferably at the most 0.5 cm$^2$, such as at the most 0.4 cm$^2$, preferably at the most 0.3 cm$^2$. Smaller coils make it possible to use a large number of coils whereas larger coils are able to provide larger fields - such as for use at a larger distance, such as for treating cells, tissue and/or micro organisms inside a container or body.

Normally, the apparatus would comprise more than 4 coils, and depending on a number of factors, the number of coils may exceed 4, 6, 8, 10, 14, 18, 20, 24 or more.

Naturally, the shape of the coils may be any shape desired. The shape of the coils will be determined on the basis of ease of manufacture, availability and requirements as to the individual positioning.

By using a number of, especially smaller, coils, the induced electric field at a given depth will have many smaller ring shaped maximum regions instead of one large ring shaped maximum region - together with a number of the desired high gradient areas. Thereby the total induced electric field will be more homogeneous and have a higher average value without eliciting any cellular activation potentials. Also the ratio between low field regions within ring shaped maxima and the total region to be treated is reduced.

The current pulses conducted in the coils preferably comprise rising and declining phases (two, three or more) resulting in the imposition of an electric field on the charges in the region to be treated. The overall duration of these events may vary depending on the pulse pattern. Thus, the events comprise increasing and declining magnetic fields that cause the appearance of temporally dependent electric fields on charged particles in particular directions in the tissue. These fields gives rise to the currents in the cells and extracellular environment consisting of moving ions and macromolecules such as proteins and nucleotides as well as amino acid, inositol phosphates and other charged signalling molecules. Thereby, cells are activated in fashion different from events such as i.e. action potentials.

Normally, each coil has a part being at least substantially perpendicular to the centre axis of the coil and being adapted to face the cells, micro organisms and/or tissue, the parts of the coils being positioned in one or more planes each comprising a plurality of coil parts.

The coils may be flat coils where the part will then be at a side surface thereof. Alternatively, the coils may be tubular, where the part would then normally be at an end portion thereof.

In order to fit in as many coils in the space as possible, it may be preferred that the parts of the coils within a predetermined area in one of the one or more planes are positioned so as to form so-called closest packing. The closest packing being a manner of packing circular elements optimally.

Also, at least one of the coils may have a part within the predetermined area has a centre to centre distance to a nearest neighbour being between 1D - 1.5D, where D is the diameter of the one coil.

Different structures may be used in order to provide as many coils as possible providing the desired gradients. In fact, the preferred structure is the above closest packing which, unfortunately, provides also adjacent coils providing fields in the same direction. Therefore, the structure according to the invention is a honeycomb structure where it is possible for all three coils adjacent to a given coil to have the opposite field than the given coil.

In one embodiment, the plurality of coils is embedded in a flat sheet of flexible material for at least partly surrounding the predetermined cells, micro organisms and/or tissue. Thus, a bendable sheet is provided for surrounding a container, a body part or the like.

Each coil may have a ratio between its inductance and resistance resulting in a pulsed current with a rise time in the range from 0.1 ms to 2 ms and a maximum current corresponding to a maximum magnetic field of 0.05-0.1 Tesla at the centre of the coil. This has been found suitable for enhancing angiogenesis in biological tissue.

Also, the pulse generating means may be adapted to generate pulses with a frequency in the range from 1 to 300 Hz, such as 10-200 Hz, preferably 20-100 Hz.

Normally, the apparatus will comprise a power supply for supplying power to the pulse generating means. Especially in order to provide a portable apparatus, the power supply may be a battery comprised within the apparatus and supplying an electric potential of 50 V or less.

Normally, the current pulses fed to the coils will be the same pulses. However, it is possible to actually provide different pulses to different coils, such as pulses having different frequencies. In that situation it is desired that those frequencies are multipla of a basic frequency in order to pulses to be provided at least substantially simultaneously.

It is preferred that the phases of the pulses have a temporal separation in order for the biochemical events to occur, and the two events are therefore normally separated by milliseconds. It has been found suitable to provide a delay of 0.01-10 ms, such as 0.05-5 ms, preferably 0.2-2 ms, such as on the order of 0.5 ms between adjacent pulses for the coils. Also, a time duration of 1-100 ms, such as 2-50 ms, preferably 5-20 ms, such as on the order of 10 ms of the pulses has been found preferred for some applications.

Also, it generally may be desired to provide a treatment over a prolonged period of time, such as a period of time exceeding 15 minutes, such as exceeding ½ hour, preferably exceeding 1 hour, such as exceeding 2 hours, such as exceeding 4 hours, preferably exceeding 10 hours, such as exceeding 15 hours, preferably exceeding 1 day, such as exceeding 2 days.

An especially preferred embodiment of the present apparatus is one being adapted to be carried by a person during operation.

In that and other embodiments, it is desired that the apparatus further comprises means for fastening the coils to a body part of a human or animal.

The present invention also relates to two preferred devices that by use of conventional CMOS IC technology create the particular currents in the coils. The devices comprises timing circuits, made from standard CMOS IC's with low power consumption. They form a free running asymmetric square wave generator that for example produces an output pulse every 18 ms with a pulse with of for example 3 ms. This pulse is applied to the output stage. The pulse pattern can consist of one or two phases. A CMOS IC, that divides the pulse frequency with 100, drives a control lamp by counting the flashes. This makes it possible to make a simple evaluation of the generator functionality and its frequency. In addition in one device, a magnetometer is incorporated to check the battery and the coils for defects. This circuit comprises a little sense coil, an amplifier, a peak rectifier and a comparator that, when beyond threshold, drives the control lamp to light permanently. The comparator threshold is passed when the stimulating coil is functioning correctly when held close to the location of the sense coil.

An alternative to the use of the sensing coil is to have the control lamp flash only when the coil current is within the limits that ensures correct functionality.

In another aspect, the invention relates to the use of the above apparatus as defined in claim 15. In this case in a preferred method the coil current is sensed.

The apparatus according to the invention may be used for enhancing tissue growth in a human or an animal, the use comprising positioning the coils adjacently to the tissue in question and operating the pulse generating means.

In this situation, the series of pulses and the coils are preferably selected so that the maximum regions of the induced electrical fields in the predetermined portion are sufficiently small in order not to elicit action potentials in living cells. Normally, a muscle cell or nerve is depolarised to an extent that the membrane potential from - 90 mV (muscle) or- 70 mV (nerve) reaches its threshold around - 55 mV whereby an action potential is elicited. Thus, the present apparatus is able to treat the cells etc. without eliciting excitable tissues.

The use comprises the step of positioning of the coils at the upper or lower jaw of the human or the animal for inducing an enhanced bone growth, such as after extraction of a tooth.

The use comprises positioning the coils at the upper or lower jaw of the human or the animal for promoting in-growth of dental implants.

The use comprises attaching the coils to a joint region of the human or the animal for treatment of arthritis or pain, and/or for promoting growth of bone and/or cartilage and/or blood vessels (angiogenesis).

the use comprises attaching two or more coils to a joint region of the human or animal to prevent arthritis or pain or to promote bone growth after a bone fracture.

The apparatus can also be used for enhancing the biochemical activity of neural tissue. Three or more coils can be attached to the head and transcranial stimulation conducted without eliciting action potentials but enhancing neural activity resulting in an increased neurosecretion and/or in cell division. This can be applied for i.e. the treatment of depression disorders.

Alternatively, the above apparatus may be used for treating micro organisms, the use comprising positioning the coils adjacently to the micro organisms in question and operating the pulse generating means.

The above apparatus may also, as a matter of fact, be used for treating seeds, plants or plant tissue. This use will comprise positioning the coils adjacently to the seeds, plants or plant tissue in question and operating the pulse generating means.

The apparatus may also be used for treating micro organisms with pulsed electromagnetic fields, the method comprising providing the above apparatus, directing the centre axes of the coils into the micro organisms, and operating the pulse generating means.

Also, it may be preferred to also have the step of providing a part of each coil, the part being at least substantially perpendicular to the centre axis of the coil and being adapted to face the cells, micro organisms and/or tissue, in one or more planes each comprising a plurality of coil parts.

The parts of the coils may be provided within a predetermined area in one of the one or more planes are positioned so as to form a closest packing.

Also, at least one of the coils having a part within the predetermined area may be provided to have a centre to centre distance to a nearest neighbour being between 1 D - 1.5D, where D is the diameter of the one coil.

In one embodiment, the plurality of coils are embedded in a flat sheet of flexible material and at least partly surrounding the predetermined cells, micro organisms and/or tissue with the flat sheet.

The use of the apparatus comprises the step of providing an even number, larger than four, of coils in sets of two, and positioning the coils of each set on at least substantially opposite sides of the cells, micro organisms and/or tissue so as to have at least substantially coincident centre axes. Again, these coils may be provided within one or more flexible sheets or e.g. within a more rigid, such as a tong like, structure.

Normally, the method would further comprise providing a power supply for supplying power to the pulse generating means. Especially for portable apparatus or for safety reasons, the power supply may be a battery comprised within the apparatus and supplying an electric potential of 50 V or less.

Depending on the actual purpose of the method, the coils and the pulse generating means may be desired to provide a series of pulses forming a temporal overlap between the varying magnetic fields from individual coils to form a periodically varying total magnetic field having a frequency in the range from 1 to 1000 Hz.

For the given pulse supplied by the pulse generating means, in each pair of coils, the first coil may conduct the current pulse in a clockwise direction and the second coil may conduct the current pulse in a counter-clockwise direction taken along the centre axes of the first and second coils, respectively, in a direction toward the cells, micro organisms and/or tissue.

The first coil of each honeycomb structure conducts the current pulse in a clockwise direction and three of the nearest neighbouring coils of the first coil conduct the current pulse in a counter clock-wise direction taken along the centre axes of the coils in a direction toward the cells, micro organisms and/or tissue.

Depending on the actual use, each coil may receive, in the series of pulses, a pulsed current with a rise time in the range from 0.1 ms to 2 ms and a maximum current adapted to provide a magnetic field of 0.01 Tesla at the centre of the coil, and the pulse generating means may generate pulses with a frequency in the range from 1 to 300 Hz.

In a number of applications, it is desired to have a number of coils, such as more than 2 coils, and depending on a number of factors, the number of coils may exceed 2, 4, 6, 8, 10, 14, 18, 20, 24 or more. In the same situation, it may be desired to then choose coils with smaller sizes than those normally used today. Therefore, the step of providing the coils preferably comprises providing coils having a cross section, perpendicularly to the centre axis, is at the most 100 $cm^2$, such as at the most 50 $cm^2$, preferably at the most 25 $cm^2$, such asat the most 10 $cm^2$, such as at the most 9 $cm^2$, preferably at the most 8 $cm^2$, such as at the most 7 $cm^2$, preferably at the most 6 $cm^2$, such as at the most 5 $cm^2$, preferably at the most 4 $cm^2$, such as at the most 3 $cm^2$, preferably at the most 2 $cm^2$, such as at the most 1 $cm^2$, preferably at the most 0.5 $cm^2$, such as at the most 0.4 $cm^2$, preferably at the most 0.3 $cm^2$.

When using the apparatus for treating human cells or tissue, the coils are fastened to a body part of a human or an animal. Alternatively, the coils may be provided fixed to e.g. a building, a wall or a bed or as a separate part, such as part of a mattress or a blanket.

Normally, the coils are positioned adjacent to the cells, micro organisms and/or tissue in question and the pulse generating means is operated.

Preferably, especially when treating cells or tissue of living beings, the series of pulses and the coils are adjusted so as for the maximum regions of the induced electrical fields in the predetermined portion to be sufficiently small in order not to elicit action potentials in living cells.

## BRIEF DESCRIPTION OF THE DRAWINGS

Not all drawings show the details of the invention. Preferred embodiments of the invention will now be described with reference to the drawings wherein:

Fig. 1A is a schematic comparison of a prior art system and an electronic diagram thereof.

Fig. 1B is an illustration of an apparatus and electronic diagrams for use in the invention.

Fig. 2 is a circuit diagram for a preferred pulse generator for use in the apparatus according to Fig. 1 B.

Fig. 3 illustrates the magnetic field lines originating from two coils positioned adjacently with oppositely directed magnetic fields.

Fig. 4A illustrates a measurement of magnetic field intensities.

Fig. 4B illustrates the magnetic field lines originating from the current pulses in a system comprising two coils as illustrated in Fig. 3.

Fig. 5 illustrates magnetic field vectors in a situation were four coils are applied as two opposite sets.

Fig. 6A illustrates a measuring of magnetic field intensities from the set-up of Fig. 5.

Fig. 6B represents the measurements from the measurement of Fig. 6A

Fig. 7 illustrates the increase in voltage of a sensing coil positioned adjacently to a single coil, the upper curve illustrates the current in the sensing coil as a function of time and the lower curve illustrates the electromotive force imposed on charged particles as a function of time.

Fig. 8 illustrates an example of a series of pulses in which + 50 V is imposed for 3 ms then followed by - 50 V for 3 ms. This results in a rapid change in the current in the coils that causes a rapidly changing magnetic field.

Fig. 9 illustrates the development of small capillaries in chicken embryos using the present invention.

Fig. 10 illustrates images taken of a chicken embryo chorioallantoic membrane with (A) or without (B) exposure for 48 hr of PEMF.

## DETAILED DESCRIPTION.

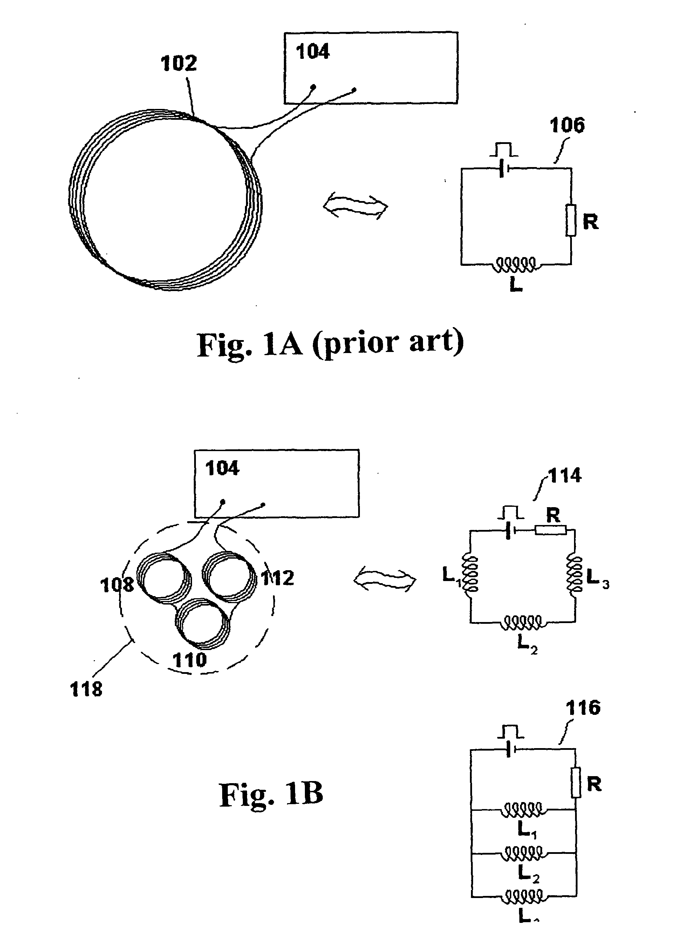

The prior art apparatus of Fig. 1A has a coil (102), having a given number of windings, and a current source 104. A schematic drawing (106) reveals its electrical properties with a current source, a resistance and an inductance.
The apparatus according to Fig. 1B comprises three coils (108, 110 and 112) imbedded in a supporting frame (118) and being connected to a current source (104). The electronic circuit (114) reveals the coil characteristics. They can be connected in series (114) and parallel (116).

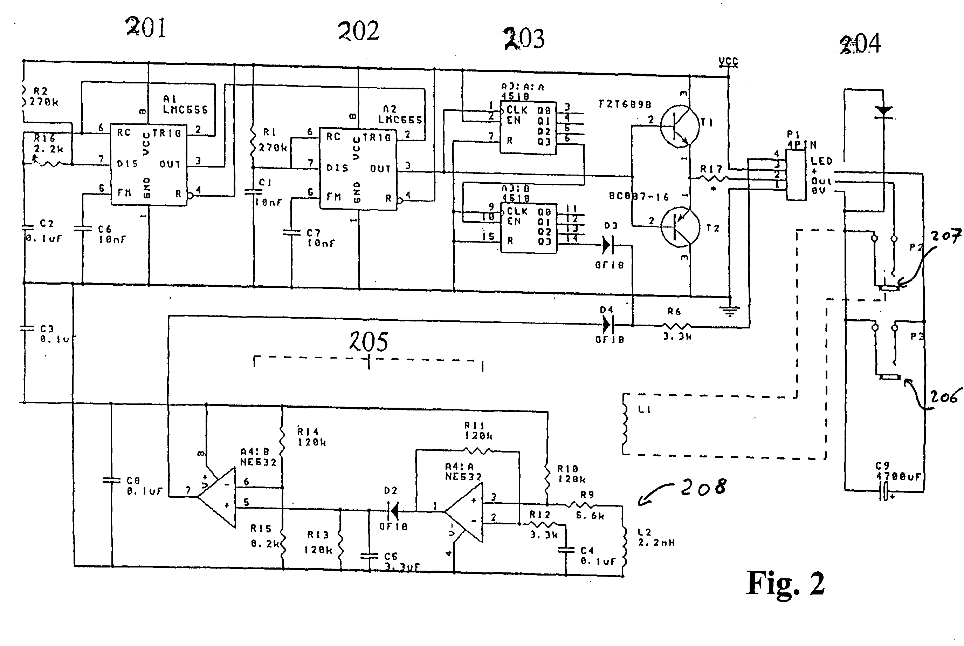

*Pulse generator (Fig. 2).*

Fig. 2 is an illustration of a preferred circuit for providing current pulses with one phase for the coils and with a sense coil system that detects when magnetic fields are created by the connected coils. This circuit is composed of a 55 Hz oscillator (201), a one-shot 3 ms circuit (202), a divider (203) 1/100 for the lamp, a front panel (204) providing output for coils (207) and external DC power (206), a magnetic field sense device (205) with a comparator on the left, a peak rectifier in the middle, and an amplifier at the right location. The circuit for the sense coil system is denoted (208).

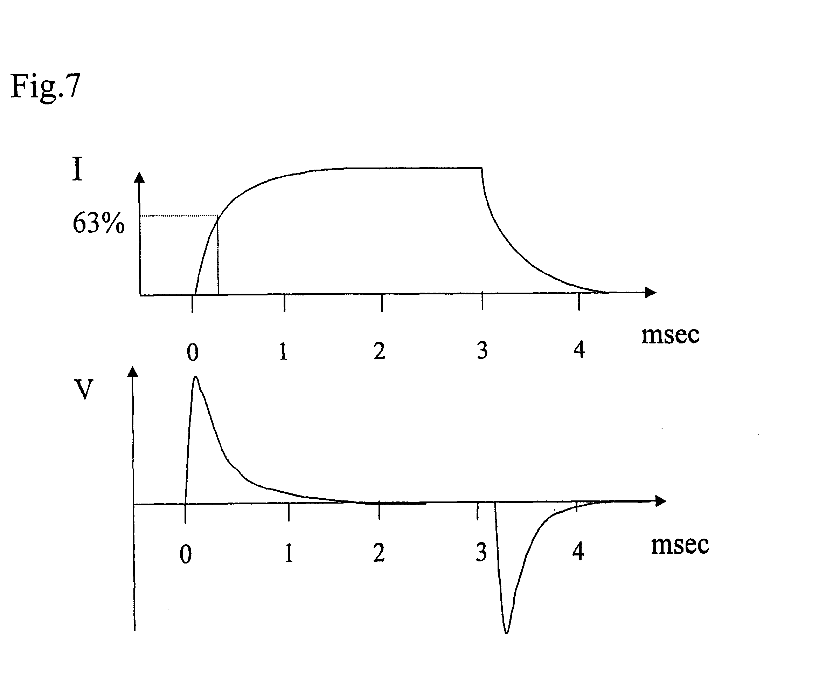

Fig. 7 illustrates measurements performed adjacently to a single coil. Illustrated is the increase in voltage in a sense coil placed underneath a single coil of the type used in this device. The rise in voltage was measured during pulse generations by means of an oscilloscope.
The upper curve illustrates the current in the coil as a function of time. The rise time for the current flow is a function of the inductance (L) and the resistance (R) of the coil circuit. As described elsewhere, the magnitude of UR (63 % of maximum current) is essential for the characteristics (duration and magnitude) of the electromotoric force induced on charged particles in the tissue.
The lower figure illustrates the electromotoric force (V) imposed on charged particles (or electrons in a wire) as a function of time. The shape of this V/ms relationship is determined by the magnitude of the slope of the A/ms relationship in the upper curve.

The necessary power for the coils is either delivered by a handheld, battery operated pulse generator (Fig. 2) or from a power source yielding up to 50 V and the sufficient amount of current. The device comprises electronic circuits, output switch, coil connector and a control lamp. The necessary power for stimulating the coils can be delivered by a handheld, battery operated pulse generator as shown in Fig.2 (or alternatively a power supply providing up to 50V DC). The device uses conventional CMOS IC technology to create the particular currents in the coils. The device comprises a timing circuit, made from standard CMOS IC's with low power consumption. It forms a free running asymmetric square wave generator that produces an output pulse i.e. every 18 ms with a pulse with of i.e. 3 ms. Those characteristics can be varied, and usually the desired frequency for pulse generation is between 1-100 Hz. The duration of the pulse will then change accordingly. This pulse is applied to the output stage, which comprises two complementary emitter followers that supply the necessary output current and is able to withstand the transients from the current switching in the coil. An output switch can be made so that it selects different resistors placed in series with the coil in order to vary the output current. A CMOS IC, that divides the pulse frequency with 100, drives a control lamp by counting the flashes. This makes it possible to make a simple evaluation of the generator functionality and its frequency. In addition, a magnetometer is incorporated to check the battery and the coils for defects. This circuit is formed by a little sense coil, an amplifier, a peak rectifier and a comparator that, when beyond threshold, drives the control lamp to be permanently on. The comparator threshold is exceeded when the stimulating coil is functioning correctly and is held close to the location of the sense coil.

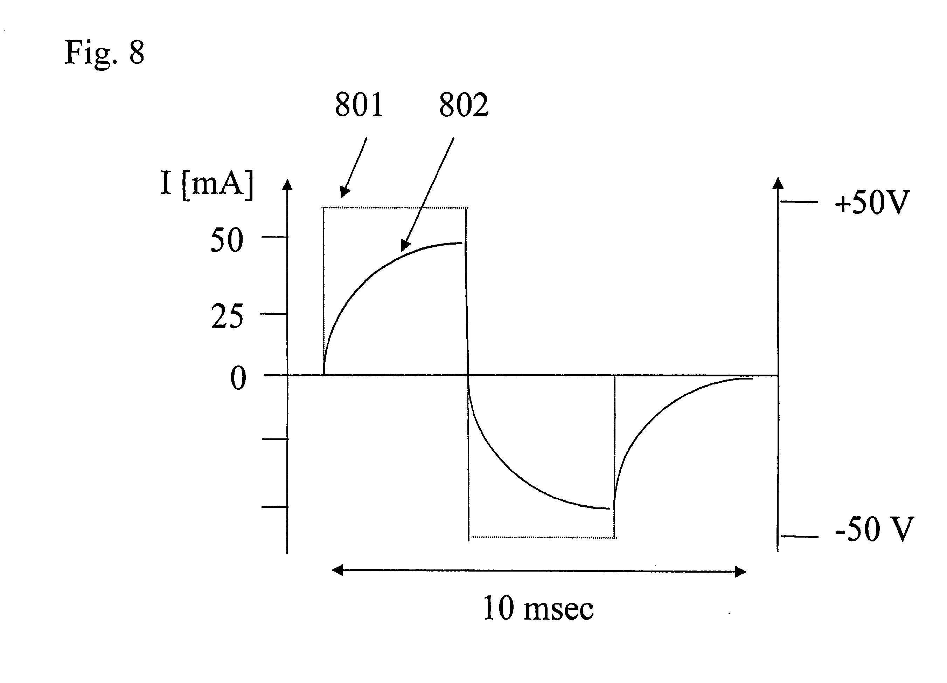

The device can also comprise a larger cabinet, electronic circuits, power lead and power switch, coil connector and control lamp. Also, a device may use conventional CMOS technology to create the voltage needed for the particular currents in the stimulating coils. The electronic circuit comprises a timing circuit that generates free running bipolar square pulse pairs. They can be produced (as in Fig. 8) every 18 ms with a duration of each pulse of 3 ms. These pulse pairs are applied to the output stage, which comprises two complementary emitter followers that supply the necessary output current and is able to withstand the transients from the current switching in the coil. By measuring the output current, a control circuit checks the stimulating coils and their connection leads. A control lamp indicates correct function by blinking with 1.8 s intervals. Fig. 8 shows and example of a series of pulses in which + 50 V is imposed for 3 ms then followed by - 50 V for 3 ms. This results in a rapid change in the current in the coils that causes a rapidly changing magnetic field.

Fig. 8 illustrates voltage applied to the coils from a pulse generator giving + and - 50 V. The increase in voltage (801) + or - 50 V drives the current (802) in the coils that consist of three phases. In the first phase + 50 V is applied, causing an increase in current with a rate constant determined by the UR ratio. After 3 ms, - 50 V is introduced and the current is reversed. This event lasts 3 ms, where after + 50 V is introduced again. After approximately 10 m the current is zero. The magnitude of the magnetic field is proportional to the current flow. The magnitude of the electromotoric force (EMF) on charged particles in the cells is proportional to the rate by which the current changes.

The full procedure in Fig. 8 lasts about 10 ms. It is highly desired that the events have a given duration in order for the biochemical events to occur. The 10 ms event can, however, be shortened up to five times by reducing the individual current transients. Thereby, the duration of the event can be as short as 2 ms. The frequency applied for sending pulses (composed of individual transients) can then i.e. vary between 2 and 500 Hz.

*Construction of coils*

The basic principles for the construction of coils are shown in Fig. 1. with the pulse generator, the resistance and inductance. When constructing coils, it is important to introduce a large potential change of at least 0.1-10 ms duration in the tissue due to the relative slow on and off rate constants for interactions in biochemical signalling. The induced electric field can be estimated by considering the electromotoric force EMF introduced into a hypothetical circuit in the tissue:

$$\text{EMF} = -\frac{1}{c}\frac{d\Phi}{dt} = -\frac{1}{c}\frac{d}{dt}\int \bar{B} \bullet d\bar{S} \, ,$$

Where B is the strength of the magnetic field and $\Phi$ is the flux of the magnetic field through the area S. Hence the induced proportional to the current flow in the coil, t, is the time and c is the speed of light.

However, there is a limit as to how rapid a rise in the voltage, and consequently the current, which causes B to rise, can be increased in the coil. This is due to the fact that the coil possesses an induction (L) as well as a resistance (R). L will limit the rate by which the current can be applied since, as the current increases, an electromotoric force of opposite direction will occur originating in the coil material.

The inductance is equal to:

$$L = 4 \pi k_m k' (n)^2 A l$$

Where L is the self inductance in Henry (V s/A), $k_m$ is a constant equal to $10^{-7}$ (Tesla m /A), n is the number of windings, A is the area, and $l$ is the length (m) of the coil.

The instantaneous electromotoric force induced is:

$$V = -L \, dI/dt$$

where I is the current (amperes) and t is the time (s).

Another important factor to consider for inducing rapid current changes in coils, is to evaluate the ratio of the inductance over the resistance in the coil.

The rate of rise of current can be evaluated by integrating the equation (Ohm's law):

$$V_1 - V_2 - L(dI/dt) = R \, I$$

Resulting in:

$$I = (V_1 - V_2)/R \, (1 - e^{-Rt/L})$$

The time it takes the current to rise is thus proportional to $\tau = UR$, where $\tau$ (measured in s) is the time it takes to reach 63 % of maximal current. Thus, if L becomes small, relative to a given R, by using fewer windings, becomes larger and the induced field, EMF = 1/c dB/dt, larger. The insertion of iron in the centre of the coil will also affect L (increase L) but the field lines will be more centred under and above the coil. Thus, coils should be constructed in such a way that L and R are matched to give the correct ratio, causing tissue currents of a sufficient magnitude and duration.

When the current is interrupted, an equally important event occurs resulting in a rapid decline in the magnetic field thus giving rise to new currents in the tissue in opposite directions. The coil material will now resist the new change in current flow and Ohm's first law gives:

$$-L \, (dI/dt) = R \, I$$

or by integration:

$$I = I_{max} \, e^{-(R/L)t}$$

Where $I_{max}$ is the original current flow before interruption, and the other symbols have their usual meaning. Thus, the current flow will stop with the time constant L/R. This factor thus determines the magnitude of current flow in the tissue when the pulse is interrupted. Surprisingly, if R is small, the duration of the current is longer. Since dB/dt thereby becomes smaller, the introduced current peak in the tissue will also be reduced. It is thus important to note the characteristics that create the waveforms that provide the driving force for ions and particles. In this embodiment, the coil positions have been uniquely matched, and the characteristics of the coils constructed in such a way that they together give the maximal effect in the tissue in the appropriate proximity.

The clinically applied pulsed electromagnetic fields normally have peak flux densities in the range of 0.1-5 mT (1-50 Gauss) with rise times in the order of hundreds of microseconds. This results in a typical dB/dt in the range of 1-50 T/s and corresponding peak induced electric fields of 0.1-1 V/m.

*Coils.*

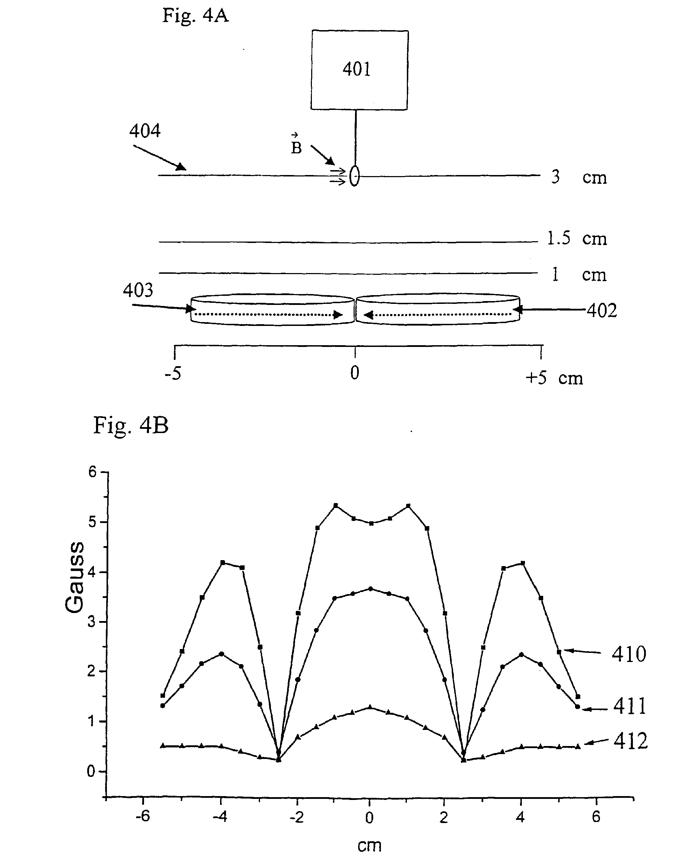

The preferred coils for treatment of i.e. osteoarthritis of the knee have a diameter of 5 cm, and a length of 2 cm. They contain 2800 windings with 0.2 mm Cu-wire and can thus be fitted to the side of the joint providing a frame for the adjacent coil technique. They have an inductance of 210 mHenry. The serial resistance of 140 (coil) + 100 Ohm (circuit) = 240 Ohm. For example UR can be: UR = 0.210/240 = $875 \cdot 10^{-6}$ s. The device has been constructed in such a way that UR can vary between 0.3 and 0.9 ms. Opposite those coils are two other coils of the same construction and with currents running in parallel with the opposite coil. Using for example 50 mA for each coil we obtain 45 Gauss in the centre or 4.5 mTesla (measured with a Gauss meter). With a rise time of half maximal magnetic force in 380 $\mu$s yields a dB/dt of 10 Tesla/s (in 0.38 ms). The induced electromotoric force in the tissue will then theoretically amount to 0.025 volts. This number will be reduced to 33 % about 2 cm away from the coil surface (see Fig. 4). Thus, the rate of increase in the magnetic field will be around 3.3 Tesla/s. Introducing iron in the centre of the coils will enhance the magnetic field, an effect that has been implemented in the coil construction.

A different set of coils have been constructed for treatment of bone growth in the jaws for patients that have been exposed for radiation therapy, for inserting implants or for promoting bone growth before the insertion of implants. Those coils have 2200 windings of 0.15 mm Cu-wire have a width of 2.5 cm and are 1 cm long. With a current of 50 mA they yield 20 Gauss in the centre. At a distance of 2 cm from the centre they yield 6.6 Gauss. Those coils are placed as two adjacent coils on the surface of the skin. The coils can be constructed and inserted i soft material in such a way that they can be strapped to the tissue for different types of treatment (enhancement of bone growth and angiogenesis, acceleration of in-growth of dental implants).

*Principle for the adjacent coil technique. Two coils.*

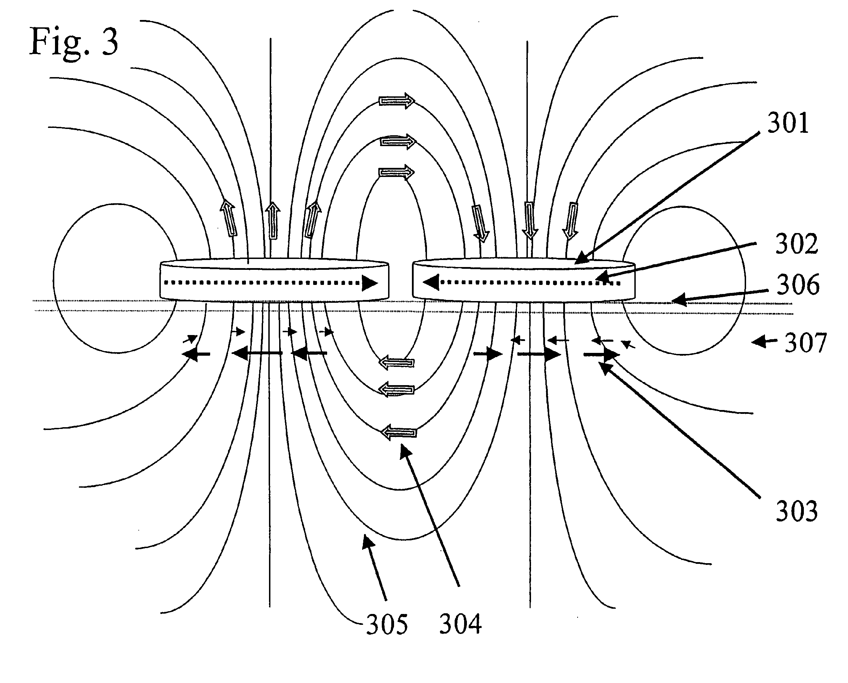

The principle for the adjacent coil technique is shown in Fig. 3. This figure illustrates the magnetic field lines originating from the current pulses in a system consisting of two coils. A coil (301) has a current in given direction (302) creating magnetic fields revealed as magnetic field lines (305) with a given magnetic field vector (304). When the current in two coils is in opposite directions as in this figure the field vectors are added together since they have the same directions in the intersection between coils. The intense currents that appear in biological tissue in the periphery and under the adjacent coils, are depicted by the filled arrows (304). In Fig. 3, the surface of the skin of the e.g. person is denoted (306) and the underlying tissue (307).

In Fig. 4A, measurements of magnetic field intensities (numerical values) along the three lines are depicted. A Gauss meter (401) (F.W. Bell, Gauss/Tesla Meter model 4048, Transverse probe model T- 4048-001 with the meter in the AC mode) was used to measure field vectors that are parallel to a line connecting the coil centres. Measurements were conducted along the lines (404, 405 and 406) that are positioned at a distance from the coils depicted in the figure.

Fig. 4B illustrates magnetic field intensities (numerical values) from the three measurements at a constant distance from the coil surface that is either 1 cm (410); 1.5 cm This figure illustrates the magnetic field lines originating from the current pulses in a system consisting of two coils. A coil (301) has a current in given direction (302) creating magnetic fields revealed as magnetic field lines (305) with a given magnetic field vector (304). When current in two coils are in opposite directions, as in this figure, the field vectors are added together since they have the same directions in the intersection between coils. The intense currents that appear in biological tissue in the periphery and under the adjacent coils are depicted by the filled arrows (304). Surface of skin (306); tissue (307). (411) or 3 cm (412). Note that at + and - 2.5 cm from coil intersection, the field vector changes its numerical value. The coils were receiving current pulses of 75 mA and a duration of 3 ms with a rise time of 0.3 ms (63 % of maximum).

With a magnetic field sensor placed perpendicular to the field vectors described above, we conducted measurements over the entire length of the two coils 1, 1.5 and 3 cm above the coils (Fig. 4B: 410,411 and 412). In the intersection area, where the coils meet, the magnetic field has its maximum value for the vector parallel to the coil axes. Above the centre of the coils, the vector attains the value 0 (as expected from the drawing Fig. 3). In the periphery (away from the intersection) the magnetic field strength rises again and has an opposite sign, (but in Fig 4B numerical values are used). When looking at the individual coils, the magnetic field lines have the highest density below and above the coil centre. The magnetic field vectors from both coils (with currents in opposite directions), are added in the intersection and therefore in this location cause relatively large magnetic fields. This can be observed when the field lines are measured parallel to the coils as shown in Fig 4B where it is evident that a large gradient appears in the tissue underneath the coils. When a third coil is added, two of the coils will have currents in the same direction and in this case a strong gradient appears at the intersections between these coils. All together when three coils are added a smaller or larger magnetic field gradient appears at different distances from the coils. This gradient provides the basis for the treatment of biological tissue. One large coil covering the same area would not provide the same size of the gradient and would therefore not be beneficial to the extent described for this invention.

*Four coils*

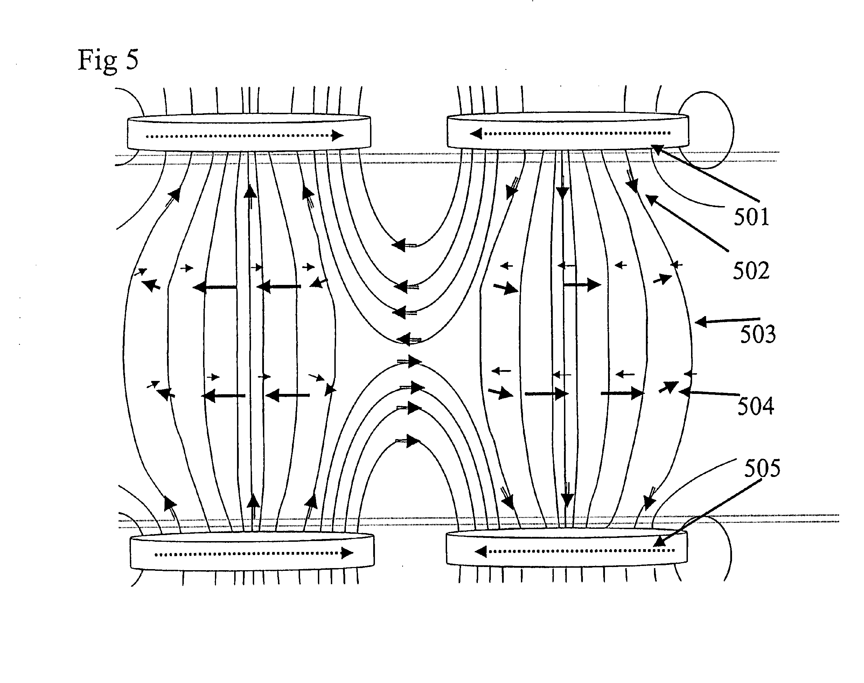

Fig. 5 illustrates magnetic field vectors in a situation were four coils are applied. Four identical coils (501) are used with currents in a given direction (505). Note that both pairs of adjacent coils have currents oriented oppositely (as in Fig. 3). The coils that are placed across each other have currents running in the same direction. Magnetic fields (503) have vectors (502) that are added in the intersection as in Fig. 4A. In Fig. 5, another gradient appears in the centre between the four coils due to the oppositely directed vectors. The filled arrows (504) show the direction of the currents in the tissue.

Magnetic field strength was measured in the space between four coils in which each pair had current in opposite directions and the opposite coil had currents in the same directions (Fig. 5). Thereby, enhanced field lines will be generated with a larger field gradient that was measured with the magnetic probe. The field line intensity was measured in the intersection between coils with the probe perpendicular to the field lines (605) measuring vectors parallel to the coil surface. In addition, line vectors perpendicular to the coils were measured at a line 2 cm from the coil centre (604). The distance was set to 10 cm, that is, the distance usually required to i.e. treatment of joints with four coils. Alternatively, this distance can be set to a smaller value giving the same type of data but being applicable for treating elbows or other small joints. Larger distances can be used for treating hips or other, larger joints.

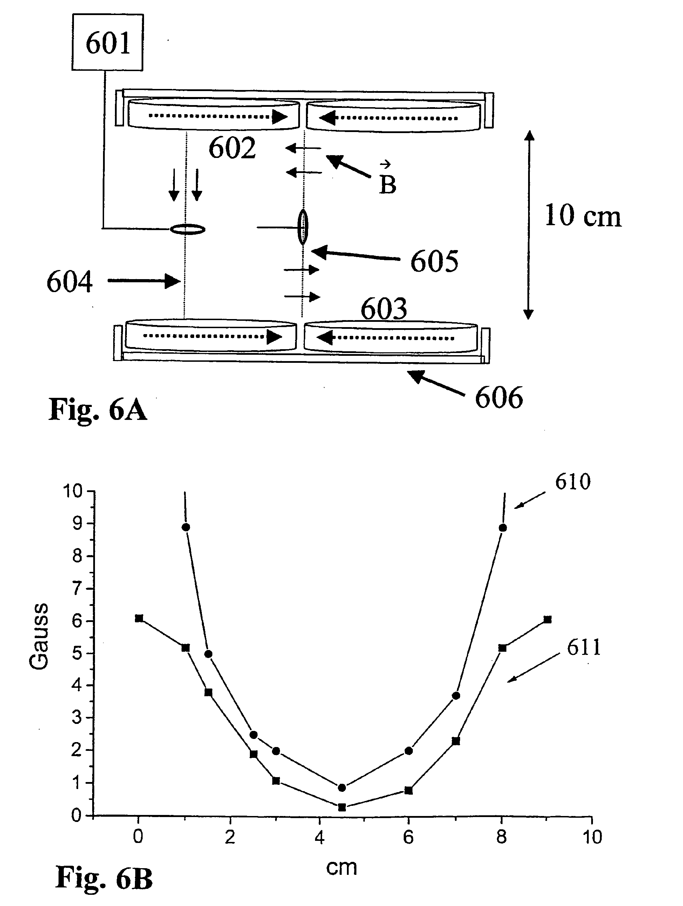

In relation to Fig. 6A, magnetic field intensities were measured originating from four coils with currents in the same direction (602,603) as illustrated also in Fig. 5. The intensities were measured by a Gauss meter with a sense coil (601) as described in Fig. 4A. Field vectors were measured along the line (604) with the coil oriented in such a way that field vectors perpendicular to the coil surface were determined. In the intersection between the four coils, the vectors parallel to the coils surface was determined along the line (605).

In this figure, indicates 606 a supporting device used for strapping the coils to the surface such as i.e. the knee or elbow.

**Fig. 6B** illustrates measurements of magnetic field intensities as described in Fig. 6A. The line (604) with vectors perpendicular to the coil surface gave intensities as shown (610) the line 605 gave field intensities depicted in 611. Each coil received 38 mA current pulses with a characteristics as in relation to Fig 4B.

Fig. 6B depicts the distance dependency. A strong magnetic field gradient appears also in the centre between the four coils (611) revealing the beneficial effect of this use of four coils with larger gradients. Alternatively, more than two coils can be used adjacent each other - i.e. 3, 4 or more. Opposite those coils could also typically be positioned coils with currents in given directions providing basis for large field gradients.

These described characteristics of line field vectors between adjacent coils are only relevant for the vectors parallel to a line combining the axes of neighbouring coils. It should, however, be emphasised that the total field strength, is a consequence of both this vector and the vector perpendicular to it describing the total field strength (%) using the equation:

$$B = \sqrt{(x^2 + y^2)},$$

where x and y are the two types of line field vectors described above.

**Description of preferred embodiments**

*Use of coils for treatment of biological tissue.*

The coil characteristics according to the invention give a new perspective to treatment with pulsed electromagnetic fields. Relatively large changes in magnetic fields can be obtained with 9-50 V using the described technology and consequently large tissue currents can be introduced with resulting beneficial biological effects (bone heeling, wound heeling, cartilage regeneration, bone growth into implants, and other types of treatments). Thus, coils can be used for a variety of treatments, some of which are now approved by the Food and Drug Administration (FDA) in the US, such as heeling of some types of bone fractures such as non-unions.

*Angiogenesis (growth of small vessels for sustaining blood circulation).*

Reduced blood circulation in the extremities is a complicating factor for a series of diseases i.e. diabetes and psoriasis. It is also seen following excessive cigarette smoking, following a high plasma cholesterol concentration and hypertension. Sustaining synthesis of new vessels is essential for repairing such damaged areas, for wound healing and for generating new blood supply to i.e. bone tissue exposed to radiation therapy. It might also be an important factor for synthesis of new bone material.

In order to characterise the effect of PEMF on angiogenesis we used the previously developed model for testing angiogenesis in chicken embryos. Three days old fertilised eggs were cracked and chicken embryos with intact yolks were placed in plastic dishes. After three days of incubation at 37 °C in 3 % $CO_2$ they were exposed to PEMF in set up using three coils for a disc with one chicken embryo. The pulse generator applied + and - 50 V with two phases (Fig. 8) and the distance between the egg and the coil surface was 4 cm. The temperature was thermostatically controlled in the incubator.

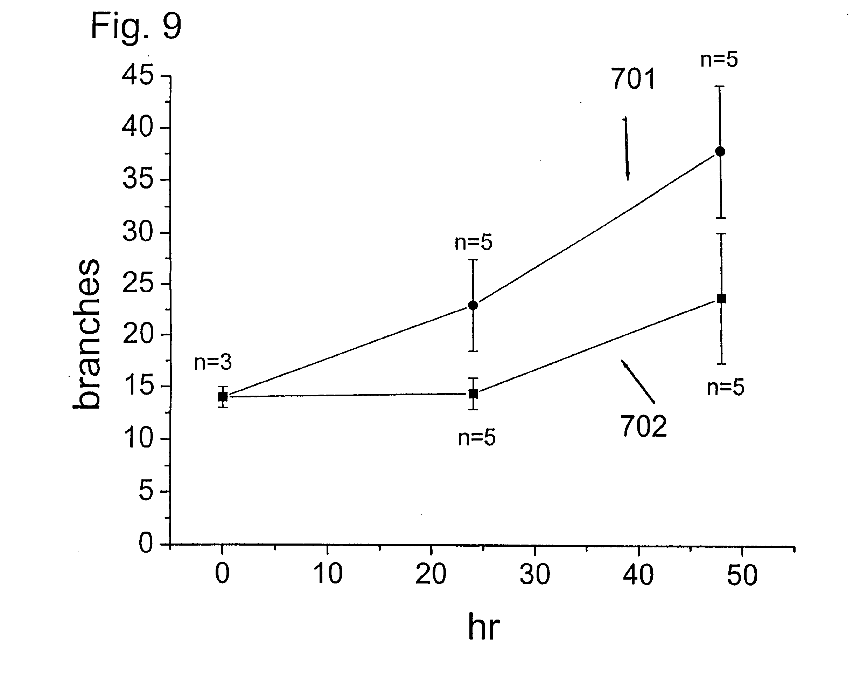

The synthesis of new blood vessels was analysed by imaging techniques. The amount of new vessels (small capillaries) of a size from 10 micrometers to several hundred micrometers were evaluated by counting the number of new branches formed (Fig. 9, 701 and 702).

Fig. 9 illustrates the number of branches at the small capillaries measured from a chicken embryo chorioallantoic membrane with and without exposure to PEMF using the device giving pulse currents as described in relation to Fig. 8. An image was taken of the membrane at the depicted time intervals and the number of branches at a 15 mm$^2$ area was counted either without (702) or with exposure to PEMF (701).

Fig.s 10A and 10B illustrate images taken of the chicken embryo chorioallantoic membrane with (A) or without (B) exposure to 48 hr of PEMF. Images were taken with a NIKON Cool Pix digital camera and images analysed by use of Adobe Photoshop software.

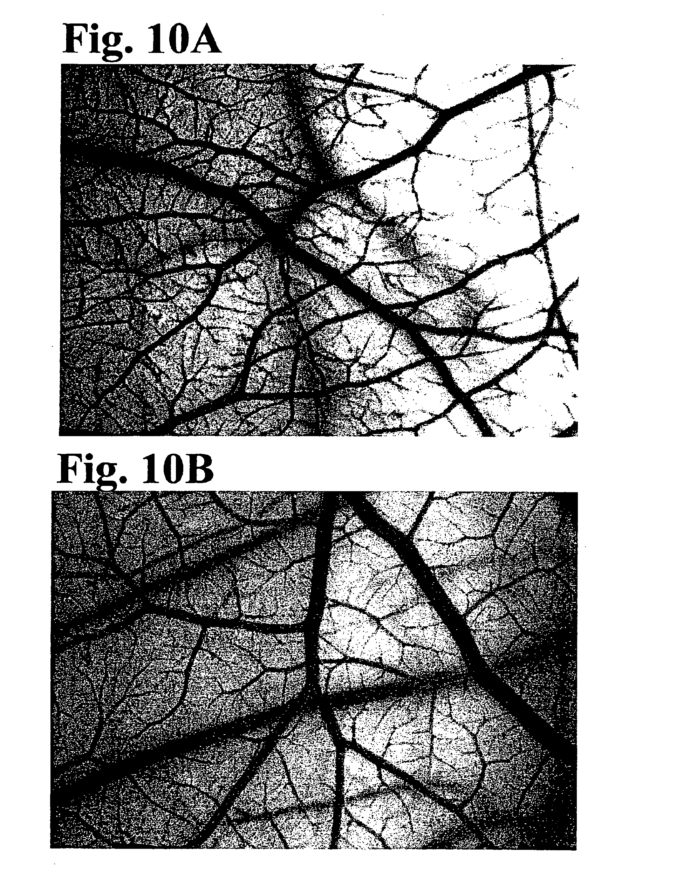

The figures 10A and 10B show that it was possible to significantly enhance the number of new vessels synthesised as well as observe an enhanced rate of organisation of the newly formed vessels (Fig. 10) using PEMF. These findings have important implications for initiating clinical research on wound healing and initiate attempts to enhance the blood circulation in patients suffering from i.e. diabetes. In addition patients with decomposed bone material due to radiation therapy can also benefit from treatment with this technology. Coils can be attached the area in question and the bone material treated.

*Treatment of joints*

The coils can be fastened to the area of treatment, i.e. the knee, where typically 4 coils are placed opposite each other as described in fig. 5. They can be fastened to the knee using Velcro© material or a different type of trapping material, and the current supplied from wire attached to the pulse generator with 9 - 50 V battery or 12 or 50 V power supply from a transformer supported by 110V or 220 V. This type of treatment can be conducted on both humans and animals, such as horses, suffering from injuries in joints.

*Treatment of dental implants*

For ingrowth of dental implants, a mask can be fitted to the jaws using 2 or more coils for each area being treated (Fig. 2). For this purpose we have constructed coils with a diameter of 25 mm described in the section: *Construction of coils.* A device can be fitted to the neck and head region of a person and thereby with an elastic material support the coils attached to the skin at the jaws. A particular problem in making implants is that after tooth extraction, there is usually a duration of several months before new bone material has grown into the area. This process can be accelerated by the above-described device. After insertion of the implant, treatment with pulsed electromagnetic fields can accelerate the growth of bone material onto the implant.

*Small fractures of the hand.*

For fractures of small bones in the hand, the small 25 mm coils (in pairs) can be used also applying Velcro© material.

*Treatment of animals*

Horses can be treated with pulsed electromagnetic fields using two or four coils as described in Figs. 2-6, by strapping coils to the joint or to the area with a bone fracture by use of Velcro© material. A pulse generator with a 12-50 V power supply or a battery can be feeding pulses to the coils and be near the animal in a stable.

*The use of the device for treatment of seeds*

Example 1. Laboratory germination

600 grams standard calibrated and polished monogerm sugar beet seeds (CV Manhattan) were imbibed in water for two hours, dubbed on filter paper and incubated in a closed plastic bag at 4°C for 17 hours. After this activation treatment, the seed lot was divided into 3 equal fractions and either left untreated or treated with PEMF for 90 min and PEMF for 240 min, followed by drying in an air stream overnight. The treated lots were then germinated in pleated paper boxes according to the ISTA-guidelines (International Rules for Seed Testing, Seed Sci. & Tech., 27, Suppl.; 1999) for sugar beet, but without pre-washing. Percent germination can be seen from table 1:

Table 1: Laboratory germination of naked sugar beet seed lots.

| Treatment | Germination, day 3 | Germination, day 4 | Germination, day 4, root length > 15mm | Germination, day 7 |
|---|---|---|---|---|
| Untreated | 39 | 91 | 21 | 96 |
| PEMF, 90 min. | 59 | 95 | 29 | 97 |
| PEMF, 240 min. | 35 | 94 | 40 | 98 |

As can be seen from table 1, PEMF treatment enhances the speed of germination of preactivated seeds in the parameter "4 day germination" with a root length of more than 15mm, indicating enhanced vigour.

Example 2. Effect of PEMF on pelleted seed.

Three monogerm non-activated sugar beet varieties, Canaria, Manhattan and Marathon were standard pelleted and coated as for the Danish marked. Equally pelleted lots were then PEMF-treated at 25V and 55V respectively. Controls were left untreated. After treatment, seed lots were analysed for laboratory germination as well as field emergence in a standard split plot design with 3 varieties, 4 replications, 200 seeds/replication.
Percent laboratory germination and final field emergence (FE) is presented in table 2: All data given is and average of the 3 varieties.

Table 2: Laboratory germination and final FE (nr) of pelleted sugar beet seed lots.

| Treatment | Germination, day 4 | Germination, day 4, root length > 15mm | Germination, day 7 | Pct. Final FE |
|---|---|---|---|---|
| Untreated control | 72 | 0 | 96 | 86,0% (100) |
| 25V PEMF, 90 min. | 79 | 5 | 98 | 86,7% (101) |
| 50V PEMF, 90 min. | 74 | 4 | 99 | 86,9% (101) |

As can be seen from table 2, PEMF-treatment of pelleted seed lots enhances the number of seeds with a root length above 15 mm after 4 days of germination.

Example 3: Effect of PEMF on field emergence of naked seed lots.

Three monogerm non-activated sugar beet varieties, Canaria, Manhattan and Marathon were PEMF-treated at 25V or 50V. Some seed lots were imbibed in water to a relative water content (RWC) on 30%, 45% and 65%, respectively, two hours before PEMF-treatment. Controls to PEMF-treatment were left untreated. After treatment, wet seed lots were air-dried. Seed lots were treated with thiram and mesurol and drilled for field emergence in a standard split plot design with 3 varieties, 4 replications, 200 seeds/replication. Successive seedling emergence was counted 3 times.
Field emergence data are given in table 3: All data given is average of 3 varieties.

Table 3: Field emergence data of naked sugar beet seed lots (HCPFE013):

| Treatment | Count1 | | Count2 | | Count 3 (final emergence) | |
|---|---|---|---|---|---|---|
| Pre-treatment | Control | PEMF 25V | Control | PEMF 25V | Control | PEMF 25V |
| Untreated | 27,9% | 28,6% (103) | 50,2% | 53,2% (106) | 80,5% | 83,8% (104) |
| 30% RWC | 29,0% | 29,7% (103) | 50,7% | 51,2% (101) | 83,2% | 82,4% (99) |
| 45% RWC | 27,8% | 29,3% (105) | 51,1% | 54,4% (107) | 82,3% | 82,2% (100) |
| 65% RWC | 29,1% | 29,4% (101) | 50,6% | 51,0% (101) | 82,5% | 81,5% (99) |
| Pre-treatment | Control | PEMF 55V | Control | PEMF 55V | Control | PEMF 55V |
| Untreated | 27,9% | 27,3% (98) | 50,2% | 51,3% (102) | 80,5% | 82,2% (102) |

As will be seen from table 3, the PEMF treatment increases the speed of emergence (Count 2). In the final emergence (Count 3) PEMF has no effect if seed lots are pre-treated, whereas the non pre-treated naked seed lots gain from PEMF-treatment.

*The use of the device for treatment of micro organisms*

Micro organisms, such as bacteria, can be treated with PEMF by which their survival can be improved under desirable conditions. It is an important technique to be able to code seeds with particular types of bacteria where after, when planted, a correct and not harmful environment supports germination and the formation of roots. Usually, the desirable bacteria are dried from containing 70 % water to have only 20 % water and subsequently be attached to the seeds. That, however, results in a strongly diminished survival rate of the bacteria, which has been a considerable problem. We have applied our apparatus, using the pulse pattern of Fig. 8, to improve the survival rate of the bacteria. It was done by exposing the bacteria for PEMF for two hours while being exposed to a procedure in which the water content was reduced from 70% to 40%. During this phase it is contemplated that particular intracellular proteins are synthesised (such as classes of heat shock proteins (hsp70)) whereby the bacteria are better withstanding the drying procedure. A subsequent addition of water and counting of colonies resulted in a 50 to 100 times better yield of bacteria when exposed to PEMF.

**Claims**

1.   An apparatus comprising:

- a plurality of electrically conducting coils (108, 110, 112), each coil having a corresponding centre axis, each centre axis being directed in operation into cells, tissue, micro organisms, seeds, plants or plant tissue,
- pulse generating means (104) operationally connected to each coil for supplying a series of current pulses for conduction in each coil, said series of pulses being adapted to generate a periodically varying magnetic field from each coil for inducing an electrical field,

wherein a number of coils are arranged in a honeycomb structure so that a first coil is arranged with three second coils adjacent to the first coil, and wherein, for a given pulse supplied by the pulse generating means, the magnetic field at the centre of the first coil is directed opposite the magnetic field at the centre of the three adjacent second coils.

2.   An apparatus according to claim 1, wherein each coil has a part being at least substantially perpendicular to the centre axis of the coil and being adapted to face the cells, tissue, micro organisms, seeds, plants or plant tissue, the parts of the coils being positioned in one or more planes each comprising a plurality of coil parts.

3.   An apparatus according to claim 1, wherein at least one of the coils has a centre to centre distance to a nearest neighbouring coil thereto in a range of 1D to 1.5D, where D is the diameter of the one coil.

4.   An apparatus according to any one of the preceding claims, wherein the plurality of coils is embedded in a flat sheet of flexible material for at least partly surrounding the cells, tissue, micro organisms, seeds, plants or plant tissue.

5.   An apparatus according to any one of the preceding claims, wherein the apparatus comprises an even number of coils arranged in sets of four, the coils of each set being adapted to be positioned on at least substantially opposite sides of the cells, tissue, micro organisms, seeds, plants or plant tissue and having at least substantially coincident centre axes.

6. An apparatus according to any one of the preceding claims, wherein each coil has a ratio between its inductance and resistance resulting in a pulsed current with a rise time in a range of 0.1 ms to 2 ms and a maximum current corresponding to a maximum magnetic field in a range of 0.05 to 0.1 Tesla at the centre of the coil.

7. An apparatus according to any one of the preceding claims, wherein the pulse generating means is adapted to generate pulses with a frequency in a range of 1 to 300 Hz.

8. An apparatus according to any one of the preceding claims, further comprising a power supply for supplying power to the pulse generating means, the power supply being a battery comprised within the apparatus and supplying an electric potential of 50 V or less.

9. An apparatus according to any one of the preceding claims, wherein the pulse generating means comprises a CMOS circuit for assisting in generating the series of current pulses.

10. An apparatus according to any one of the preceding claims, wherein the coils and the pulse generating means are operable to provide a series of pulses forming a temporal overlap between the varying magnetic fields from individual coils to form a periodically varying total magnetic field having a frequency in a range of 1 to 1000 Hz.

11. An apparatus according to any of the preceding claims, wherein a cross section of each coil, perpendicularly to the centre axis, is at most 100 $cm^2$, such as at most 50 $cm^2$, preferably at most 25 $cm^2$, such as at most 10 $cm^2$, such as at most 9 $cm^2$, preferably at most 8 $cm^2$, such as at most 7 $cm^2$, preferably at most 6 $cm^2$, such as at most 5 $cm^2$, preferably at most 4 $cm^2$, such as at most 3 $cm^2$, preferably at most 2 $cm^2$, such as at most 1 $cm^2$, preferably at most 0.5 $cm^2$, such as at most 0.4 $cm^2$, preferably at most 0.3 $cm^2$.

12. An apparatus according to any one of the preceding claims, wherein the pulse generating means and the coils are adapted to induce electrical fields in a predetermined portion of the apparatus, which electrical fields are sufficiently small in order not to elicit action potentials in living cells.

13. An apparatus according to any one of the preceding claims, wherein the pulse generating means is operable to provide a delay in a range of 0.01 to 10 ms, such as in a range of 0.05 to 5 ms, preferably in a range of 0.2 to 2 ms, such as in the order of 0.5 ms between adjacent pulses for the coils.

14. An apparatus according to any one of claims 1 to 12, wherein the pulse generating means is operable to provide pulses having a time duration in a range of 1 to 100 ms, such as in a range of 2 to 50 ms, preferably in a range of 5 to 20 ms, such as in the order of 10 ms.

15. An apparatus according to any one of claims 1 to 14, the apparatus being adapted to operate for a period of time exceeding 15 minutes, such as exceeding ½ hour, such as exceeding 1 hour, such as a period of time exceeding 2 hours, such as exceeding 4 hours, preferably exceeding 10 hours, such as exceeding 15 hours, preferably exceeding 1 day, such as exceeding 2 days.

16. A method for stimulating and/or modulating seeds, plants or plant tissue with pulsating electromagnetic fields, the method comprising steps of:

- providing a plurality of coils (108, 110, 112), each coil having a corresponding centre axis directed into the seeds, plants or plant tissue,
- providing a series of current pulses to each coil, said series of pulses being operable to generate a periodically varying magnetic field from each coil, and
- arranging the coils in a honeycomb structure with a first coil thereof having three adjacent coils so that for a given current pulse, a magnetic field from the first coil is directed oppositely to magnetic fields from the three adjacent coils thereby creating substantial magnetic field gradients in the seeds, plants or plant tissue.

17. A method according to claim 16, further comprising the step of providing a part of each coil, the part being at least substantially perpendicular to the centre axis of the coil and being orientated to face the seeds, plants or plant tissue, in one or more planes each comprising a plurality of coil parts.

18. A method according to any one of claims 16 to 17, wherein the coils are arranged so that at least one of the coils has a centre to centre distance to a nearest neighbouring coil thereto in a range of 1D - 1.5D, where D is the diameter of the one coil.

19. A method according to any one of claims 16 to 18, comprising steps of:

   (a) embedding the plurality of coils in a flat sheet of flexible material; and
   (b) at least partly surrounding the seeds, plants or plant tissue with the flat sheet.

20. A method according to any one of claims 16 to 19, wherein the providing step comprises providing an even number of coils in sets of two coils, and positioning the coils of each set on at least substantially opposite sides of the seeds, plants or plant tissue so that the coils of each set have at least substantially coincident centre axes.

21. A method according to any one of claims 16 to 20, wherein each coil receives, in the series of pulses, each pulse having a pulsed current with a rise time in a range of 0.1 ms to 2 ms and a maximum current operable to provide a magnetic field of 0.01 Tesla at the centre of the coil.

22. A method according to any one of claims 16 to 21, wherein the pulse generating means generates pulses having a frequency in a range of 1 to 300 Hz.

23. A method according to any one of claims 16 to 22, further comprising a step of providing a power supply for supplying power to the pulse generating means, the power supply being a battery comprised within the apparatus and supplying an electric potential of 50 V or less.

24. A method according to any one of claims 16 to 23, wherein the coils and the pulse generating means are operable to provide a series of pulses forming a temporal overlap between the varying magnetic fields from individual coils to form a periodically varying total magnetic field having a frequency in a range of 1 to 1000 Hz.

25. A method according to any one of claims 16 to 24, wherein the step of providing the coils comprises providing coils having a cross section, perpendicularly to the centre axis, which is at most 100 $cm^2$, such as at most 50 $cm^2$, preferably at most 25 $cm^2$, such as at most 10 $cm^2$, such as at most 9 $cm^2$, preferably at most 8 $cm^2$, such as at most 7 $cm^2$, preferably at most 6 $cm^2$, such as at most 5 $cm^2$, preferably at most 4 $cm^2$, such as at most 3 $cm^2$, preferably at most 2 $cm^2$, such as at most 1 $cm^2$, preferably at most 0.5 $cm^2$, such as at most 0.4 $cm^2$, preferably at most 0.3 $cm^2$.

26. A method according to any one of claims 16 to 25, further comprising a step of providing a delay in a range of 0.01 to 10 ms, such as in a range of 0.05 to 5 ms, preferably in a range of 0.2 to 2 ms, such as in the order of 0.5 ms between adjacent pulses for the coils.

27. A method according to any one of claims 16 to 26, further comprising the step of providing pulses having a time duration in a range of 1 to 100 ms, such as in a range of 2 to 50 ms, preferably in a range of 5 to 20 ms, such as in the order of 10 ms.

28. A method according to any one of claims 16 to 27, the method comprising a step of providing pulses for the coils for a period of time exceeding 15 minutes, such as exceeding ½ hour, such as exceeding 1 hour, such as exceeding 2 hours, such as exceeding 4 hours, preferably exceeding 10 hours, such as exceeding 15 hours, preferably exceeding 1 day, such as exceeding 2 days.

**Patentansprüche**

1. Vorrichtung, umfassend:

   - eine Vielzahl elektrischer leitfähiger Spulen (108, 110, 112), wobei jede Spule eine entsprechende Mittelachse hat, wobei jede Mittelachse bei Betätigung in Zellen, Gewebe, Mikroorganismen, Saatgut, Pflanzen oder Pflanzengewebe eingeführt wird,

- pulserzeugende Mittel (104), die bei Betätigung mit jeder Spule für die Bereitstellung einer Strömungspulsserie zur Leitung in jeder Spule verbunden sind, wobei die Pulsserie dafür vorgesehen ist, aus jeder Spule ein periodisch variierendes magnetisches Feld zur Induzierung eines elektrischen Felds zu erzeugen,

worin eine Anzahl von Spulen in einer Bienenwabenstruktur angeordnet sind, so dass eine erste Spule mit drei an der ersten Spule anliegenden zweiten Spulen angeordnet ist, und worin, für einen bestimmten durch die pulserzeugenden Mittel bereitgestellten Puls, das magnetische Feld im Zentrum der ersten Spule dem magnetischen Feld im Zentrum der drei anliegenden zweiten Spulen entgegengesetzt geführt ist.

2. Vorrichtung nach Anspruch 1, worin jede Spule einen mindestens im Wesentlichen senkrecht zur Mittelachse der Spule verlaufenden Teil besitzt, der dazu eingerichtet ist, den Zellen, dem Gewebe, den Mikroorganismen, dem Saatgut, den Pflanzen oder dem Pflanzengewebe gegenüberzuliegen, wobei die Teile der Spulen in einer oder mehreren Ebene(n) angebracht sind, die jede eine Vielzahl von Spulenteilen umfassen.

3. Vorrichtung nach Anspruch 1, worin mindestens eine der Spulen ein Zentrum besitzt, wobei der Zentrumabstand zur nächstgelegenen Nachbarspule in einem Bereich von 1D bis 1,5 D liegt, indem D der Durchmesser der einen Spule ist.

4. Vorrichtung nach irgendeinem der vorhergehenden Ansprüche, worin die Vielzahl von Spulen in einem flachen Blech aus flexiblem Material eingebettet ist, um mindestens teilweise die Zellen, das Gewebe, die Mikroorganismen, das Saatgut, die Pflanzen oder das Pflanzengewebe zu umschliessen.

5. Vorrichtung nach einem der vorhergehenden Ansprüche, worin die Vorrichtung eine gerade Zahl von in 4 Sätzen angeordneten Spulen umfasst, wobei die Spulen eines jeden Satzes dazu eingerichtet sind, um an den wenigstens im Wesentlichen entgegengesetzten Seiten der Zellen, des Gewebes, der Mikroorganismen, des Saatguts, der Pflanzen oder des Pflanzengewebes angebracht zu sein und wenigstens im Wesentlichen übereinstimmende Mittelachsen zu haben.

6. Vorrichtung nach irgendeinem der vorhergehenden Ansprüche, worin jede Spule ein Verhältnis zwischen seiner Induktivität und Resistenz hat, welches zu einer impulsförmigen Strömung mit einer Anstiegszeit in einem Bereich von 0,1 ms bis 2 ms und einer Maximalströmung, die einem maximalen magnetischen Feld in einem Verhältnis von 0,05 bis 0,1 Tesla in der Mitte der Spule entspricht, führt.

7. Vorrichtung nach irgendeinem der vorhergehenden Ansprüche, worin die pulserzeugenden Mittel dazu eingerichtet sind, Pulse mit einer Frequenz in einem Bereich von 1 bis 300 Hz zu erzeugen.

8. Vorrichtung nach irgendeinem der vorhergehenden Ansprüche, die weiterhin eine Energieversorgung umfasst, um den pulserzeugenden Mitteln Energie zuzuführen, wobei die Energieversorgung eine in der Vorrichtung enthaltende Batterie ist und eine elektrische Leistung von 50 V oder weniger bereitstellt.

9. Vorrichtung nach irgendeinem der vorgehenden Ansprüche, worin die pulserzeugenden Mittel einen CMOS-Kreislauf umfassen, um bei der Erzeugung der Strömungspulsserie mitzuwirken.

10. Vorrichtung nach irgendeinem der vorhergehenden Ansprüche, worin die Spulen und die pulserzeugenden Mittel bedient werden, um eine Pulsserie, die eine zeitliche Überlappung zwischen den varrierenden magnetischen Feldern individueller Spulen darstellen, bereitzustellen, um ein periodisch variierendes gesamtes Magnetfeld, das eine Frequenz in einem Bereich von 1 bis 1000 Hz hat, zu schaffen.

11. Vorrichtung nach irgendeinem der vorhergehenden Ansprüche, worin ein Querschnitt jeder Spule senkrecht zur Mittelachse höchstens 100 cm$^2$, wie zum Beispiel höchstens 50 cm$^2$, vorzugsweise höchstens 25 cm$^2$, wie zum Beispiel höchstens 10 cm$^2$, wie zum Beispiel höchstens 9 cm$^2$, vorzugsweise höchstens 8 cm$^2$, wie zum Beispiel höchstens 7 cm$^2$, vorzugsweise höchstens 6 cm$^2$, wie zum Beispiel höchstens 5 cm$^2$, vorzugsweise höchstens 4 cm$^2$, wie zum Beispiel höchstens 3 cm$^2$, vorzugsweise höchstens 2 cm$^2$, wie zum Beispiel höchstens 1 cm$^2$, vorzugsweise höchstens 0,5 cm$^2$, wie zum Beispiel höchstens 0,4 cm$^2$, vorzugsweise höchstens 0,3 cm$^2$, hat.

12. Vorrichtung nach irgendeinem der vorhergehenden Ansprüche, worin die pulserzeugenden Mittel und die Spulen dazu eingerichtet sind, elektrische Felder in einer vorherbestimmten Menge der Vorrichtung zu erzeugen, die ausreichend klein sind, um in lebenden Zellen kein Aktionspotential auszulösen.

13. Vorrichtung nach irgendeinem der vorhergehenden Ansprüche, worin die pulserzeugenden Mittel bedient werden, um eine Verzögerung im Bereich von 0,01 bis 10 ms, wie zum Beispiel im Bereich von 0,05 bis 5 ms, vorzugsweise im Bereich von 0,2 bis 2 ms, wie zum Beispiel in der Reihenfolge von 0,5 ms zwischen anliegendenden Pulsen der Spulen, zu erzeugen.

14. Vorrichtung nach irgendeinen der Ansprüche 1 bis 12, worin die pulserzeugenden Mittel bedient werden, um Pulse mit einer Zeitdauer in einem Bereich von 1 bis 100 ms, wie zum Beispiel in einem Bereich von 2 bis 50 ms, vorzugsweise in einem Bereich von 5 bis 20 ms, wie in der Reihenfolge von 10 ms, bereitzustellen.

15. Vorrichtung nach irgendeinem der Ansprüche 1 bis 14, wobei die Vorrichtung dazu eingerichtet ist, in einem Zeitraum von mehr als 15 Minuten, wie zum Beispiel mehr als 1/2 Stunde, wie zum Beispel mehr als 1 Stunde, wie zum Beispiel einem Zeitraum von mehr als 2 Stunden, wie zum Beispiel mehr als 4 Stunden, vorzugsweise mehr als 10 Stunden, wie zum Beispiel mehr als 15 Stunden, vorzugsweise mehr als 1 Tag, wie zum Beispiel mehr als 2 Tagen zu arbeiten.

16. Verfahren zur Stimulierung und/oder Anpassung von Saatgut, Pflanzen oder Pflanzengewebe mit pulsierenden elektromagnetischen Feldern, wobei das Verfahren die Schritte umfasst:

   - Bereitstellung einer Vielfalt von Spulen (108, 110, 112), wobei jede Spule eine auf Saatgut, Pflanzen oder Pflanzengewebe gezielte entsprechende Mittelachse hat,
   - Bereitstellung einer Serie von Strömungspulsen zu jeder Spule, wobei die Pulsserie bedient wird, um aus jeder Spule ein periodisch variierendes magnetisches Feld zu erzeugen, und
   - Aufstellung der Spulen in einer Bienenwabenstruktur, wo eine erste Spule davon drei anliegende Spulen hat, so dass für einen bestimmten Strömungspuls ein magnetisches Feld von einer ersten Spule entgegengesetzt zu magnetischen Feldern der drei anliegenden Spulen geführt wird, wodurch erhebliche magnetische Feldgradienten im Saatgut, den Pflanzen oder dem Pflanzengewebe erzeugt werden.

17. Verfahren nach Anspruch 16, das weiterhin den Schritt zur Bereitstellung eines mindestens im Wesentlichen senkrecht zur Mittelachse der Spule verlaufende Teils jeder Spule umfasst, die ausgerichtet ist, dem Saatgut, den Pflanzen und dem Pflanzengewebe in einer oder mehreren Ebenen gegenüberzuliegen, die jede eine Vielzahl von Spulenteilen umfassen.

18. Verfahren nach irgendeinem der Ansprüche 16 bis 17, worin die Spulen so angeordnet sind, dass mindestens eine der Spulen ein Zentrum besitzt, wobei der Zentrumabstand zur dichtesten Nachbarspule in einem Bereich von 1D - 1,5 D liegt, indem D der Durchmesser der einen Spule ist.

19. Verfahren nach irgendeinem der Ansprüche 16 bis 18, umfassend die Schritte:

   (a) Einbettung der Vielzahl von Spulen in einem flachen Blech aus flexiblem Material; und
   (b) mindestens teilweise Umgebung des Saatguts, der Pflanzen oder des Pflanzengewebes mit dem flachen Blech.

20. Verfahren nach irgendeinem der Ansprüche 16 bis 19, worin der Bereitstellungsschritt die Bereitstellung einer geraden Zahl von Spulen in zwei Spulensätzen umfasst, und die Spulen jedes Satzes an mindestens im Wesentlichen entgegengesetzten Seiten des Saatguts, der Pflanzen oder des Pflanzengewebes angebracht sind, so dass die Spulen jeden Sets mindestens im Wesentlichen übereinstimmende Mittelachsen haben.

21. Verfahren nach irgendeinem der Ansprüche 16 bis 20, worin jede Spule in der Pulsserie erhalten wird, wobei jeder Puls einen Pulsstrom mit einer Anstiegszeit in einem Bereich von 0,1 ms bis 2 ms und einem Maximalstrom, der betätigt wird, ein magnetisches Feld von 0,01 Tesla im Zentrum der Spule bereitzustellen, besitzt.

22. Verfahren nach irgendeinem der Ansprüche 16 bis 21, worin die pulserzeugenden Mittel Pulse erzeugen, die eine Frequenz in einem Bereich von 1 bis 300 Hz haben.

23. Verfahren nach irgendeinem der Ansprüche 16 bis 22, die weiterhin einen Schritt zur Bereitstellung einer Energieversorgung umfassen, um den pulserzeugenden Mitteln Energie zuzuführen, wobei die Energieversorgung eine in der Vorrichtung enthaltende Batterie ist und eine elektrische Leistung von 50 V oder weniger bereitstellt.

24. Verfahren nach irgendeinem der Ansprüche 16 bis 23, worin die Spulen und die pulserzeugenden Mittel betätigt werden können, um eine Serie von Pulsen, die eine zeitliche Überlappung zwischen den variierenden magnetischen Feldern individueller Spulen darstellen, bereitzustellen, um zeitliche varüerende totale Magnetfelder, die eine Frequenz in einem Bereich von 1 bis 1000 Hz haben, zu schaffen.

25. Verfahren nach irgendeinem der Ansprüche 16 bis 24, worin der Schritt zur Bereitstellung der Spulen die Bereitstellung von Spulen mit einem Querschnitt senkrecht zur Mittelachse umfasst, die höchstens 100 $cm^2$, wie zum Beispiel höchstens 50 $cm^2$, vorzugsweise höchstens 25 $cm^2$, wie zum Beispiel höchstens 10 $cm^2$, wie zum Beispiel höchstens 9 $cm^2$, vorzugsweise höchstens 8 $cm^2$, wie zum Beispiel höchstens 7 $cm^2$, vorzugsweise höchstens 6 $cm^2$, wie zum Beispiel höchstens 5 $cm^2$, vorzugsweise höchstens 4 $cm^2$, wie zum Beispiel höchstens 3 $cm^2$, vorzugsweise höchstens 2 $cm^2$, wie zum Beispiel höchstens 1 $cm^2$, vorzugsweise höchstens 0,5 $cm^2$, wie zum Beispiel höchstens 0,4 $cm^2$, vorzugsweise höchstens 0,3 $cm^2$, ist.

26. Verfahren nach irgendeinem der Ansprüche 16 bis 25, das weiterhin einen Schritt zur Erzeugung einer Verzögerung im Bereich von 0,01 bis 10 ms, wie zum Beispiel im Bereich von 0,05 bis 5 ms, vorzugsweise im Bereich von 0,2 bis 2 ms, wie zum Beispiel in der Reihenfolge von 0,5 ms, zwischen anliegenden Pulsen der Spulen umfasst.

27. Verfahren nach irgendeinen der Ansprüche 16 bis 26, dass weiterhin den Schritt zur Bereitstellung von Pulsen in einer Zeitdauer in einem Bereich von 1 bis 100 ms, wie zum Beispiel in einem Bereich von 2 bis 50 ms, vorzugsweise in einem Bereich von 5 bis 20 ms, wie zum Beispiel in der Reihenfolge von 10 ms, umfasst.

28. Verfahren nach irgendeinem der Ansprüche 16 bis 27, das einen Schritt zur Bereitstellung von Pulsen für die Spulen in einem Zeitraum von mehr als 15 Minuten, wie zum Beispiel mehr als 1/2 Stunde, wie zum Beispiel mehr als 1 Stunde, wie zum Beispiel mehr als 2 Stunden, wie zum Beispiel mehr als 4 Stunden, vorzugsweise mehr als 10 Stunden, wie zum Beispiel mehr als 15 Stunden, vorzugsweise mehr als 1 Tag, wie zum Beispiel mehr als 2 Tagen, umfasst.

**Revendications**

1. Appareil comprenant :

   - une pluralité de bobines électriquement conductrices (108, 110, 112), chaque bobine ayant un axe central correspondant, chaque axe central étant dirigé en fonctionnement à l'intérieur de cellules, de tissus, de microorganismes, de graines, de plantes ou de tissus végétaux,
   - un moyen générateur d'impulsions (104) connecté de manière opérationnelle à chaque bobine pour fournir une série d'impulsions de courant pour la conduction dans chaque bobine, ladite série d'impulsions étant adaptée pour générer un champ magnétique variant périodiquement à partir de chaque bobine pour induire un champ électrique,

   où un nombre de bobines sont arrangées dans une structure en nid d'abeille de manière à ce qu'une première bobine soit arrangée avec trois secondes bobines adjacentes à la première bobine, et où, pour une impulsion donnée fournie par le moyen générateur d'impulsions, le champ magnétique au centre de la première bobine est dirigé de manière opposée au champ magnétique au centre des trois secondes bobines adjacentes.

2. Appareil selon la revendication 1, où chaque bobine a une partie étant au moins essentiellement perpendiculaire à l'axe central de la bobine et étant adaptée pour faire face aux cellules, tissus, microorganismes, graines, plantes ou tissus végétaux, les parties des bobines étant positionnées dans un ou plusieurs plans, chacun comprenant une pluralité de parties de bobines.

3. Appareil selon la revendication 1, où au moins l'une des bobines a une distance centre à centre jusqu'à une bobine la plus proche l'avoisinant dans une gamme de 1D à 1,5D, où D est le diamètre d'une bobine seule.

4. Appareil selon une quelconque des revendications précédentes, où la pluralité de bobines est encastrée dans une feuille plate faite d'un matériau flexible pour entourer au moins en partie les cellules, tissus, microorganismes, graines, plantes
ou tissus végétaux.

**5.** Appareil selon une quelconque des revendications précédentes, où l'appareil comprend un nombre pair de bobines arrangées par groupes de quatre, les bobines de chaque groupe étant adaptées pour être positionnées sur des côtés au moins essentiellement opposés des cellules, tissus, microorganismes, graines, plantes ou tissus végétaux, et ayant des axes centraux coïncidant au moins essentiellement.

**6.** Appareil selon une quelconque des revendications précédentes, où chaque bobine a un rapport entre son inductance et sa résistance résultant en un courant d'impulsion avec un temps de montée dans une gamme de 0,1 ms à 2 ms et un courant maximum correspondant à un champ magnétique maximum dans une gamme de 0,05 à 0,1 Tesla au centre de la bobine.

**7.** Appareil selon une quelconque des revendications précédentes, où le moyen générateur d'impulsions est adapté pour générer des impulsions avec une fréquence dans une gamme de 1 à 300 Hz.

**8.** Appareil selon une quelconque des revendications précédentes, comprenant en outre une source d'alimentation électrique pour alimenter le moyen générateur d'impulsions en électricité, la source d'alimentation électrique étant une pile comprise à l'intérieur de l'appareil et fournissant un potentiel électrique de 50V ou moins.

**9.** Appareil selon une quelconque des revendications précédentes, où le moyen générateur d'impulsions comprend un circuit CMOS pour assister à la génération des séries d'impulsions de courant.

**10.** Appareil selon une quelconque des revendications précédentes, où l'on peut faire fonctionner les bobines et le moyen générateur d'impulsions afin qu'ils fournissent une série d'impulsions formant un chevauchement temporel entre les champs magnétiques variables issus des bobines individuelles pour former un champ magnétique total variant périodiquement ayant une fréquence dans une gamme de 1 à 1000 Hz.

**11.** Appareil selon une quelconque des revendications précédentes, où une section transversale de chaque bobine, perpendiculairement à l'axe central, est d'au plus 100 cm$^2$, par exemple d'au plus 50 cm$^2$, de préférence d'au plus 25 cm$^2$, par exemple d'au plus 10 cm$^2$, par exemple d'au plus 9 cm$^2$, de préférence d'au plus 8 cm$^2$, par exemple d'au plus 7 cm$^2$, de préférence d'au plus 6 cm$^2$, par exemple d'au plus 5 cm$^2$, de préférence d'au plus 4 cm$^2$, par exemple d'au plus 3 cm$^2$, de préférence d'au plus 2 cm$^2$, par exemple d'au plus 1 cm$^2$, de préférence d'au plus 0,5 cm$^2$, par exemple d'au plus 0,4 cm$^2$, de préférence d'au plus 0,3 cm$^2$.

**12.** Appareil selon une quelconque des revendications précédentes, où le moyen générateur d'impulsions et les bobines sont adaptés pour induire des champs électriques dans une portion prédéterminée de l'appareil, lesquels champs électriques sont suffisamment petits pour ne pas engendrer de potentiels d'action dans des cellules vivantes.

**13.** Appareil selon une quelconque des revendications précédentes, où l'on peut faire fonctionner le moyen générateur d'impulsions afin qu'il fournisse un retard dans une gamme de 0,01 à 10 ms, par exemple dans une gamme de 0,05 à 5 ms, de préférence dans une gamme de 0,2 à 2 ms, par exemple de l'ordre de 0,5 ms, entre des impulsions adjacentes pour les bobines.

**14.** Appareil selon une quelconque des revendications 1 à 12, où l'on peut faire fonctionner le moyen générateur d'impulsions afin qu'il fournisse des impulsions ayant une durée de temps dans une gamme de 1 à 100 ms, par exemple dans une gamme de 2 à 50 ms, de préférence dans une gamme de 5 à 20 ms, par exemple de l'ordre de 10 ms.

**15.** Appareil selon une quelconque des revendications 1 à 14, l'appareil étant adapté pour fonctionner pendant une période de temps excédant 15 minutes, par exemple excédant ½ heure, par exemple excédant 1 heure, par exemple une période de temps excédant 2 heures, par exemple excédant 4 heures, de préférence excédant 10 heures, par exemple excédant 15 heures, de préférence excédant 1 journée, par exemple excédant 2 journées.

**16.** Méthode pour la stimulation et/ou la modulation de graines, de plantes ou de tissus végétaux avec des champs électromagnétiques pulsatoires, la méthode comprenant les étapes consistant à :

- fournir une pluralité de bobines (108, 110, 112), chaque bobine ayant un axe central correspondant et dirigé à l'intérieur des graines, plantes ou tissus végétaux,
- fournir une série d'impulsions de courant à chaque bobine, où l'on peut faire fonctionner ladite série d'impulsions afin qu'elle génère un champ magnétique variant périodiquement à partir de chaque bobine, et

**EP 1 216 076 B1**

- arranger les bobines dans une structure en nid d'abeille avec une première bobine de celle-ci ayant trois bobines adjacentes de manière à ce que, pour une impulsion de courant donnée, un champ magnétique issu de la première bobine est dirigé de manière opposée aux champs magnétiques issus des trois bobines adjacentes, créant ainsi des gradients considérables de champ magnétique dans les graines, plantes ou tissus végétaux.

**17.** Méthode selon la revendication 16, comprenant en outre l'étape consistant à fournir une partie de chaque bobine, la partie étant au moins essentiellement perpendiculaire à l'axe central de la bobine et étant orientée de manière à faire face aux graines, plantes ou tissus végétaux, dans un ou plusieurs plans, chacun comprenant une pluralité de parties de bobines.

**18.** Méthode selon une quelconque des revendications 16 à 17, où les bobines sont arrangées de manière à ce qu'au moins l'une des bobines a une distance centre à centre jusqu'à une bobine la plus proche l'avoisinant dans une gamme de 1D - 1,5D, où D est le diamètre de l'une des bobines seule.

**19.** Méthode selon une quelconque des revendications 16 à 18, comprenant les étapes consistant à :

(a) encastrer la pluralité de bobines dans une feuille plate faite d'un matériau flexible ; et
(b) entourer au moins partiellement les graines, plantes ou tissus végétaux avec la feuille plate.

**20.** Méthode selon une quelconque des revendications 16 à 19, où l'étape consistant à fournir comprend fournir un nombre pair de bobines par groupes de deux bobines, et positionner les bobines de chaque groupe sur des côtés au moins essentiellement opposés des graines, plantes ou tissus végétaux de manière à ce que les bobines de chaque groupe aient des axes centraux coïncidant au moins essentiellement.

**21.** Méthode selon une quelconque des revendications 16 à 20, où chaque bobine reçoit, dans le série d'impulsions, chaque impulsion ayant un courant d'impulsion avec un temps de montée dans une gamme de 0,1 ms à 2 ms et un courant maximum que l'on peut faire fonctionner afin qu'il fournisse un champ magnétique de 0,01 Tesla au centre de la bobine.

**22.** Méthode selon une quelconque des revendications 16 à 21, où le moyen générateur d'impulsions génère des impulsions ayant une fréquence dans une gamme de 1 à 300 Hz.

**23.** Méthode selon une quelconque des revendications 16 à 22, comprenant en outre une étape consistant à fournir une source d'alimentation électrique pour alimenter le moyen générateur d'impulsions en électricité, la source d'alimentation électrique étant une pile comprise à l'intérieur de l'appareil et fournissant un potentiel électrique de 50 V ou moins.

**24.** Méthode selon une quelconque des revendications 16 à 23, où l'on peut faire fonctionner les bobines et le moyen générateur d'impulsions afin qu'ils fournissent une série d'impulsions formant un chevauchement temporel entre les champs magnétiques variables issus des bobines individuelles pour former un champ magnétique total variant périodiquement ayant une fréquence dans une gamme de 1 à 1000 Hz.

**25.** Méthode selon une quelconque des revendications 16 à 24, où l'étape consistant à fournir les bobines comprend fournir des bobines ayant une section transversale, perpendiculairement à l'axe central, qui soit d'au plus 100 $cm^2$, par exemple d'au plus 50 $cm^2$, de préférence d'au plus 25 $cm^2$, par exemple d'au plus 10 $cm^2$, par exemple d'au plus 9 $cm^2$, de préférence d'au plus 8 $cm^2$, par exemple d'au plus 7 $cm^2$, de préférence d'au plus 6 $cm^2$, par exemple d'au plus 5 $cm^2$, de préférence d'au plus 4 $cm^2$, par exemple d'au plus 3 $cm^2$, de préférence d'au plus 2 $cm^2$, par exemple d'au plus 1 $cm^2$, de préférence d'au plus 0,5 $cm^2$, par exemple d'au plus 0,4 $cm^2$, de préférence d'au plus 0,3 $cm^2$.

**26.** Méthode selon une quelconque des revendications 16 à 25, comprenant en outre une étape consistant à fournir un retard dans une gamme de 0,01 à 10 ms, par exemple dans une gamme de 0,05 à 5 ms, de préférence dans une gamme de 0,2 à 2 ms, par exemple de l'ordre de 0,5 ms, entre des impulsions adjacentes pour les bobines.

**27.** Méthode selon une quelconque des revendications 16 à 26, comprenant en outre l'étape consistant à fournir des impulsions ayant une durée de temps dans une gamme de 1 à 100 ms, par exemple dans une gamme de 2 à 50 ms, de préférence dans une gamme de 5 à 20 ms, par exemple de l'ordre de 10 ms.

**28.** Méthode selon une quelconque des revendications 16 à 27, la méthode comprenant une étape consistant à fournir des impulsions pour les bobines pour une période de temps excédant 15 minutes, par exemple excédant ½ heure, par exemple excédant 1 heure, par exemple excédant 2 heures, par exemple excédant 4 heures, de préférence excédant 10 heures, par exemple excédant 15 heures, de préférence excédant 1 journée, par exemple excédant 2 journées.

Fig. 1A (prior art)

Fig. 1B

**Fig. 2**

EP 1 216 076 B1

Fig. 3

301

302

306

307

303

305  304

EP 1 216 076 B1

Fig. 4A

Fig. 4B

Fig 5

501
502
503
504
505

**Fig. 6A**

**Fig. 6B**

Fig.7

EP 1 216 076 B1

Fig. 8

Fig. 9

## Fig. 10A

## Fig. 10B